# EUROPEAN PATENT APPLICATION

(11) **EP 3 998 277 A1**
(43) Date of publication of application: **18.05.2022**
(21) Application number: 20837520.4
(22) Date of filing: 07.07.2020
(51) Int. Cl.: C07K 14/54, A61K 38/00, A61K 47/68, A61P 37/00

(54) **NOVEL IL-10 VARIANT PROTEIN AND USE THEREOF**

(30) Priority: 08.07.2019 KR 20190082149
(71) Applicant: Progen Co., Ltd., Seoul 06591 (KR); Genexine, Inc., Seongnam-si, Gyeonggi-do 13488 (KR)
(72) Inventor: SHIN, Eunju, Yongin-si, Gyeonggi-do 16862 (KR); NAM, Eunjoo, Seoul 05501 (KR)
(74) Representative: Dehns
(86) International application number: PCT/KR2020/008871
(87) International publication number: WO 2021/006605

(57) **Abstract**

The present invention relates to a novel IL-10 variant protein and use thereof, and more particularly, to a novel IL-10 variant protein whose immune stimulatory activity is inhibited and yield of production is increased by forming monomers when expressed.

<110>    PROGEN CO., LTD.
         Genexine, Inc.

<120>    A novel IL-10 variant protein and use thereof

<130>    PT20-5202

<150>    KR 10-2019-0082149
<151>    2019-07-08

<160>    46

<170>    KoPatentIn 3.0

<210>    1
<211>    169
<212>    PRT
<213>    Artificial Sequence

<220>
<223>    monomeric IL-10 variant


<400>    1
Ser Pro Gly Gln Gly Thr Gln Ser Glu Asn Ser Cys Thr His Phe Pro
  1               5                  10                  15

Gly Asn Leu Pro Asn Met Leu Arg Asp Leu Arg Asp Ala Phe Ser Arg
             20                  25                  30

Val Lys Thr Phe Phe Gln Met Lys Asp Gln Leu Asp Asn Leu Leu Leu
         35                  40                  45

Lys Glu Ser Leu Leu Glu Asp Phe Lys Gly Tyr Leu Gly Cys Gln Ala
     50                  55                  60

Leu Ser Glu Met Ile Gln Phe Tyr Leu Glu Glu Val Met Pro Gln Ala
 65                  70                  75                  80

Glu Asn Gln Asp Pro Asp Ile Lys Ala His Val Asn Ser Leu Gly Glu
                 85                  90                  95

Asn Leu Lys Thr Leu Arg Leu Arg Leu Arg Arg Cys His Arg Phe Leu
            100                 105                 110

Pro Cys Glu Asn Gly Gly Ser Gly Gly Ser Gly Gly Ser Lys Ser Lys
        115                 120                 125

Ala Val Glu Gln Val Lys Asn Ala Phe Asn Lys Leu Gln Glu Lys Gly
    130                 135                 140

Ile Tyr Lys Ala Met Ser Glu Phe Asp Ile Phe Ile Asn Tyr Ile Glu
145                 150                 155                 160

Ala Tyr Met Thr Met Lys Ile Arg Asn
                165


<210>    2
<211>    20
<212>    PRT
<213>    Artificial Sequence

<220>
<223>    linker peptide

<400>    2
Ala Ala Gly Ser Gly Gly Gly Gly Gly Ser Gly Gly Gly Gly Ser Gly
1               5                   10                  15

Gly Gly Gly Ser
            20

<210>    3
<211>    5
<212>    PRT
<213>    Artificial Sequence

<220>
<223>    linker peptide

<400>    3
Gly Gly Ser Gly Gly
1               5

<210>    4
<211>    9
<212>    PRT
<213>    Artificial Sequence

<220>
<223>    spacer peptide

<400>    4
Gly Gly Ser Gly Gly Ser Gly Gly Ser
1               5

<210>    5
<211>    6
<212>    PRT
<213>    Artificial Sequence

<220>
<223>    spacer peptide

<400>    5
Gly Gly Gly Ser Gly Gly
1               5

<210>    6
<211>    214
<212>    PRT
<213>    Artificial Sequence

<220>
<223>    modified Fc region lysine deleted

<400>    6
Ser His Thr Gln Pro Leu Gly Val Phe Leu Phe Pro Pro Lys Pro Lys
1               5                   10                  15

Asp Thr Leu Met Ile Ser Arg Thr Pro Glu Val Thr Cys Val Val Val
            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg
            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Met His Glu Ala Leu His Asn His Tyr Thr Gln Lys Ser
        195                 200                 205

Leu Ser Leu Ser Leu Gly
    210


<210>    7
<211>    215
<212>    PRT
<213>    Artificial Sequence

<220>
<223>    modified Fc region


<400>    7
Ser His Thr Gln Pro Leu Gly Val Phe Leu Phe Pro Pro Lys Pro Lys
1               5                   10                  15

Asp Thr Leu Met Ile Ser Arg Thr Pro Glu Val Thr Cys Val Val Val
            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg
            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Met His Glu Ala Leu His Asn His Tyr Thr Gln Lys Ser
        195                 200                 205

Leu Ser Leu Ser Leu Gly Lys
    210                 215


<210>   8
<211>   214
<212>   PRT
<213>   Artificial Sequence

<220>
<223>   modified Fc region lysine deleted


<400>   8
Ser His Thr Gln Pro Leu Gly Val Phe Leu Phe Pro Pro Lys Pro Lys
1               5                   10                  15

Asp Gln Leu Met Ile Ser Arg Thr Pro Glu Val Thr Cys Val Val Val
            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg

            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Leu His Glu Ala Leu His Asn His Tyr Thr Gln Lys Ser
        195                 200                 205

Leu Ser Leu Ser Leu Gly
    210

<210> 9
<211> 215
<212> PRT
<213> Artificial Sequence

<220>
<223> modified Fc region

<400> 9
Ser His Thr Gln Pro Leu Gly Val Phe Leu Phe Pro Pro Lys Pro Lys
1               5                   10                  15

Asp Gln Leu Met Ile Ser Arg Thr Pro Glu Val Thr Cys Val Val Val
            20                  25                  30

Asp Val Ser Gln Glu Asp Pro Glu Val Gln Phe Asn Trp Tyr Val Asp
        35                  40                  45

Gly Val Glu Val His Asn Ala Lys Thr Lys Pro Arg Glu Glu Gln Phe
    50                  55                  60

Asn Ser Thr Tyr Arg Val Val Ser Val Leu Thr Val Leu His Gln Asp
65                  70                  75                  80

Trp Leu Asn Gly Lys Glu Tyr Lys Cys Lys Val Ser Asn Lys Gly Leu
                85                  90                  95

Pro Ser Ser Ile Glu Lys Thr Ile Ser Lys Ala Lys Gly Gln Pro Arg
            100                 105                 110

Glu Pro Gln Val Tyr Thr Leu Pro Pro Ser Gln Glu Glu Met Thr Lys
        115                 120                 125

Asn Gln Val Ser Leu Thr Cys Leu Val Lys Gly Phe Tyr Pro Ser Asp
    130                 135                 140

Ile Ala Val Glu Trp Glu Ser Asn Gly Gln Pro Glu Asn Asn Tyr Lys
145                 150                 155                 160

Thr Thr Pro Pro Val Leu Asp Ser Asp Gly Ser Phe Phe Leu Tyr Ser
                165                 170                 175

Arg Leu Thr Val Asp Lys Ser Arg Trp Gln Glu Gly Asn Val Phe Ser
            180                 185                 190

Cys Ser Val Leu His Glu Ala Leu His Asn His Tyr Thr Gln Lys Ser
        195                 200                 205

Leu Ser Leu Ser Leu Gly Lys
    210                 215


<210>    10
<211>    160
<212>    PRT
<213>    Artificial Sequence

<220>
<223>    dimeric IL-10 I87A variant


<400>    10
Ser Pro Gly Gln Gly Thr Gln Ser Glu Asn Ser Cys Thr His Phe Pro
 1               5                  10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ala Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Lys Ser Lys Ala Val Glu Gln Val Lys Asn Ala Phe
        115                 120                 125

Asn Lys Leu Gln Glu Lys Gly Ile Tyr Lys Ala Met Ser Glu Phe Asp
    130                 135                 140

Ile Phe Ile Asn Tyr Ile Glu Ala Tyr Met Thr Met Lys Ile Arg Asn
145                 150                 155                 160


<210>    11
<211>    166
<212>    PRT
<213>    Artificial Sequence

<220>
<223>    prior monomeric IL-10 I87A variant

<400>    11
Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ala Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Gly Gly Gly Ser Gly Gly Lys Ser Lys Ala Val Glu
        115                 120                 125

Gln Val Lys Asn Ala Phe Asn Lys Leu Gln Glu Lys Gly Ile Tyr Lys
    130                 135                 140

Ala Met Ser Glu Phe Asp Ile Phe Ile Asn Tyr Ile Glu Ala Tyr Met
145                 150                 155                 160

Thr Met Lys Ile Arg Asn
                165


<210>    12
<211>    5
<212>    PRT
<213>    Artificial Sequence

<220>
<223>    linker peptide


<400>    12
Gly Gly Gly Gly Ser
1               5


<210>    13
<211>    6
<212>    PRT
<213>    Artificial Sequence

<220>
<223>    linker peptide


<400>    13
Gly Ser Ser Gly Gly Ser
1               5

<210>    14
<211>    18
<212>    PRT
<213>    Artificial Sequence

<220>
<223>    linker peptide


<400>    14
Lys Glu Ser Gly Ser Val Ser Ser Glu Gln Leu Ala Gln Phe Arg Ser
1               5                   10                  15

Leu Asp




<210>    15
<211>    14
<212>    PRT
<213>    Artificial Sequence

<220>
<223>    linker peptide


<400>    15
Glu Gly Lys Ser Ser Gly Ser Gly Ser Glu Ser Lys Ser Thr
1               5                   10


<210>    16
<211>    12
<212>    PRT
<213>    Artificial Sequence

<220>
<223>    linker peptide


<400>    16
Gly Ser Ala Gly Ser Ala Ala Gly Ser Gly Glu Phe
1               5                   10


<210>    17
<211>    5
<212>    PRT
<213>    Artificial Sequence

<220>
<223>    linker peptide


<400>    17
Glu Ala Ala Ala Lys
1               5


<210>    18
<211>    12
<212>    PRT

<213>    Artificial Sequence

<220>
<223>    linker peptide


<400>    18
Cys Arg Arg Arg Arg Arg Arg Glu Ala Glu Ala Cys
1               5                   10


<210>    19
<211>    46
<212>    PRT
<213>    Artificial Sequence

<220>
<223>    linker peptide


<400>    19
Ala Glu Ala Ala Ala Lys Glu Ala Ala Ala Lys Glu Ala Ala Ala Lys
1               5                   10                  15

Glu Ala Ala Ala Lys Ala Leu Glu Ala Glu Ala Ala Ala Lys Glu Ala
            20                  25                  30

Ala Ala Lys Glu Ala Ala Ala Lys Glu Ala Ala Ala Lys Ala
        35                  40                  45



<210>    20
<211>    8
<212>    PRT
<213>    Artificial Sequence

<220>
<223>    linker peptide


<400>    20
Gly Gly Gly Gly Gly Gly Gly Gly
1               5


<210>    21
<211>    6
<212>    PRT
<213>    Artificial Sequence

<220>
<223>    linker peptide


<400>    21
Gly Gly Gly Gly Gly Gly
1               5


<210>    22
<211>    13
<212>    PRT

<213>    Artificial Sequence

<220>
<223>    linker peptide


<400>    22
Ala Glu Ala Ala Ala Lys Glu Ala Ala Ala Ala Lys Ala
1               5                   10


<210>    23
<211>    5
<212>    PRT
<213>    Artificial Sequence

<220>
<223>    linker peptide


<400>    23
Pro Ala Pro Ala Pro
1               5


<210>    24
<211>    17
<212>    PRT
<213>    Artificial Sequence

<220>
<223>    linker peptide


<400>    24
Val Ser Gln Thr Ser Lys Leu Thr Arg Ala Glu Thr Val Phe Pro Asp
1               5                   10                  15

Val



<210>    25
<211>    6
<212>    PRT
<213>    Artificial Sequence

<220>
<223>    linker peptide


<400>    25
Pro Leu Gly Leu Trp Ala
1               5


<210>    26
<211>    10
<212>    PRT
<213>    Artificial Sequence

<220>
<223>    linker peptide

<400>    26
Thr Arg His Arg Gln Pro Arg Gly Trp Glu
1               5                   10

<210>    27
<211>    10
<212>    PRT
<213>    Artificial Sequence

<220>
<223>    linker peptide

<400>    27
Ala Gly Asn Arg Val Arg Arg Ser Val Gly
1               5                   10

<210>    28
<211>    8
<212>    PRT
<213>    Artificial Sequence

<220>
<223>    linker peptide

<400>    28
Arg Arg Arg Arg Arg Arg Arg Arg
1               5

<210>    29
<211>    4
<212>    PRT
<213>    Artificial Sequence

<220>
<223>    linker peptide

<400>    29
Gly Phe Leu Gly
1

<210>    30
<211>    31
<212>    PRT
<213>    Artificial Sequence

<220>
<223>    linker peptide

<400>    30
Gly Ser Ser Gly Gly Ser Gly Ser Ser Gly Gly Ser Gly Gly Gly Asp
1               5                   10                  15

Glu Ala Asp Gly Ser Arg Gly Ser Gln Lys Ala Gly Val Asp Glu

20                  25                  30

<210>   31
<211>   30
<212>   PRT
<213>   Artificial Sequence

<220>
<223>   linker peptide

<400>   31
Gly Gly Gly Gly Ser Gly Gly Gly Gly Ser Gly Gly Gly Gly Ser Glu
1               5                   10                  15

Pro Lys Ser Ser Asp Lys Thr His Thr Cys Pro Pro Cys Pro
            20                  25                  30

<210>   32
<211>   25
<212>   PRT
<213>   Artificial Sequence

<220>
<223>   tPA signal peptide

<400>   32
Met Asp Ala Met Leu Arg Gly Leu Cys Cys Val Leu Leu Leu Cys Gly
1               5                   10                  15

Ala Val Phe Val Ser Pro Ser His Ala
            20                  25

<210>   33
<211>   180
<212>   PRT
<213>   Artificial Sequence

<220>
<223>   FceRIa

<400>   33
Val Pro Gln Lys Pro Lys Val Ser Leu Asn Pro Pro Trp Asn Arg Ile
1               5                   10                  15

Phe Lys Gly Glu Asn Val Thr Leu Thr Cys Asn Gly Asn Asn Phe Phe
            20                  25                  30

Glu Val Ser Ser Thr Lys Trp Phe His Asn Gly Ser Leu Ser Glu Glu
        35                  40                  45

Thr Asn Ser Ser Leu Asn Ile Val Asn Ala Lys Phe Glu Asp Ser Gly
    50                  55                  60

Glu Tyr Lys Cys Gln His Gln Gln Val Asn Glu Ser Glu Pro Val Tyr
65                  70                  75                  80

Leu Glu Val Phe Ser Asp Trp Leu Leu Leu Gln Ala Ser Ala Glu Val
                    85                  90                  95

Val Met Glu Gly Gln Pro Leu Phe Leu Arg Cys His Gly Trp Arg Asn
            100                 105                 110

Trp Asp Val Tyr Lys Val Ile Tyr Tyr Lys Asp Gly Glu Ala Leu Lys
        115                 120                 125

Tyr Trp Tyr Glu Asn His Asn Ile Ser Ile Thr Asn Ala Thr Val Glu
    130                 135                 140

Asp Ser Gly Thr Tyr Tyr Cys Thr Gly Lys Val Trp Gln Leu Asp Tyr
145                 150                 155                 160

Glu Ser Glu Pro Leu Asn Ile Thr Val Ile Lys Ala Pro Arg Glu Lys
                165                 170                 175

Tyr Trp Leu Gln
            180


<210>    34
<211>    15
<212>    PRT
<213>    Artificial Sequence

<220>
<223>    linker peptide


<400>    34
Gly Gly Gly Gly Ser Gly Gly Gly Gly Ser Gly Gly Gly Gly Ser
  1               5                  10                  15


<210>    35
<211>    33
<212>    PRT
<213>    Artificial Sequence

<220>
<223>    linker peptide


<400>    35
Gly Gly Gly Gly Ser Gly Gly Gly Gly Ser Gly Gly Gly Gly Ser Glu
  1               5                  10                  15

Lys Glu Lys Glu Glu Gln Glu Glu Arg Thr His Thr Cys Pro Pro Cys
             20                  25                  30

Pro



<210>    36
<211>    30
<212>    PRT
<213>    Artificial Sequence

<220>
<223>    linker peptide

<400>    36
Arg Asn Thr Gly Arg Gly Gly Glu Glu Lys Lys Gly Ser Lys Glu Lys
1               5                   10                  15

Glu Glu Gln Glu Glu Arg Glu Thr Lys Thr Pro Glu Cys Pro
            20                  25                  30


<210>    37
<211>    30
<212>    PRT
<213>    Artificial Sequence

<220>
<223>    linker peptide


<400>    37
Gly Gly Gly Gly Ser Gly Gly Gly Gly Ser Gly Gly Gly Gly Ser Glu
1               5                   10                  15

Pro Lys Ser Cys Asp Lys Thr His Thr Cys Pro Pro Cys Pro
            20                  25                  30


<210>    38
<211>    26
<212>    PRT
<213>    Artificial Sequence

<220>
<223>    linker peptide


<400>    38
Gly Ser Gly Gly Gly Ser Gly Thr Leu Val Thr Val Ser Ser Glu Ser
1               5                   10                  15

Lys Tyr Gly Pro Pro Cys Pro Pro Cys Pro
            20                  25


<210>    39
<211>    169
<212>    PRT
<213>    Artificial Sequence

<220>
<223>    monomeric IL-10 I87A variant


<400>    39
Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
50                  55                  60

Leu Ser Glu Met Ile Gln Phe Tyr Leu Glu Glu Val Met Pro Gln Ala
65                  70                  75                  80

Glu Asn Gln Asp Pro Asp Ala Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Gly Gly Ser Gly Gly Ser Gly Gly Ser Lys Ser Lys
        115                 120                 125

Ala Val Glu Gln Val Lys Asn Ala Phe Asn Lys Leu Gln Glu Lys Gly
    130                 135                 140

Ile Tyr Lys Ala Met Ser Glu Phe Asp Ile Phe Ile Asn Tyr Ile Glu
145                 150                 155                 160

Ala Tyr Met Thr Met Lys Ile Arg Asn
                165

<210>    40
<211>    15
<212>    PRT
<213>    Artificial Sequence

<220>
<223>    linker peptide

<400>    40
Glu Pro Lys Ser Ser Asp Lys Thr His Thr Cys Pro Pro Cys Pro
1               5                   10                  15

<210>    41
<211>    15
<212>    PRT
<213>    Artificial Sequence

<220>
<223>    linker peptide

<400>    41
Glu Pro Lys Ser Cys Asp Lys Thr His Thr Cys Pro Pro Cys Pro
1               5                   10                  15

<210>    42
<211>    8
<212>    PRT
<213>    Artificial Sequence

<220>
<223>    linker peptide

<400>    42

Thr His Thr Cys Pro Pro Cys Pro
1               5

<210>    43
<211>    55
<212>    PRT
<213>    Artificial Sequence

<220>
<223>    linker peptide

<400>    43
Gly Gly Gly Gly Ser Gly Gly Gly Gly Ser Gly Gly Gly Gly Ser Ala
1               5                   10                  15

Lys Asn Thr Thr Ala Pro Ala Thr Thr Arg Asn Thr Thr Arg Gly Gly
            20                  25                  30

Glu Glu Lys Lys Lys Glu Lys Glu Lys Glu Glu Gln Glu Glu Arg Thr
        35                  40                  45

His Thr Cys Pro Pro Cys Pro
    50                  55

<210>    44
<211>    19
<212>    PRT
<213>    Artificial Sequence

<220>
<223>    linker peptide

<400>    44
Ala Gly Ser Gly Gly Gly Gly Gly Ser Gly Gly Gly Gly Ser Gly Gly
1               5                   10                  15

Gly Gly Ser

<210>    45
<211>    15
<212>    PRT
<213>    Artificial Sequence

<220>
<223>    linker peptide

<400>    45
Gly Gly Gly Ser Gly Gly Ser Thr His Thr Cys Pro Pro Cys Pro
1               5                   10                  15

<210>    46
<211>    160
<212>    PRT
<213>    Artificial Sequence

<220>
<223>    wild type human IL-10 (mature form)


<400>    46
Ser Pro Gly Gln Gly Thr Gln Ser Glu Asn Ser Cys Thr His Phe Pro
  1               5                  10                  15

Gly Asn Leu Pro Asn Met Leu Arg Asp Leu Arg Asp Ala Phe Ser Arg
             20                  25                  30

Val Lys Thr Phe Phe Gln Met Lys Asp Gln Leu Asp Asn Leu Leu Leu
         35                  40                  45

Lys Glu Ser Leu Leu Glu Asp Phe Lys Gly Tyr Leu Gly Cys Gln Ala
     50                  55                  60

Leu Ser Glu Met Ile Gln Phe Tyr Leu Glu Glu Val Met Pro Gln Ala
 65                  70                  75                  80

Glu Asn Gln Asp Pro Asp Ile Lys Ala His Val Asn Ser Leu Gly Glu
                 85                  90                  95

Asn Leu Lys Thr Leu Arg Leu Arg Leu Arg Arg Cys His Arg Phe Leu
            100                 105                 110

Pro Cys Glu Asn Lys Ser Lys Ala Val Glu Gln Val Lys Asn Ala Phe
        115                 120                 125

Asn Lys Leu Gln Glu Lys Gly Ile Tyr Lys Ala Met Ser Glu Phe Asp
    130                 135                 140

Ile Phe Ile Asn Tyr Ile Glu Ala Tyr Met Thr Met Lys Ile Arg Asn
145                 150                 155                 160

## Description

### TECHNICAL FIELD

The present invention is drawn to a novel IL-10 variant protein and use thereof. Particularly, the present invention is drawn to a novel IL-10 variant protein whose yield of production and immunosuppressive activity is enhanced and use thereof.

### BACKGROUND ART

Interleukin 10 (hereinafter referred to as "IL-10") is an anti-inflammatory cytokine expressed as a non-covalently bound homodimer of about 37 kDa called cytokine synthesis inhibitory factor (CSIF). IL-10 plays an important role in the induction and maintenance of immune tolerance, and these dominant anti-inflammatory properties have been known for a long time. IL-10 inhibits the secretion of pro-inflammatory cytokines such as TNFα, IL-1, IL-6, and IL-12 as well as Th1 cytokines such as IL-2 and INFγ, and regulate the differentiation and proliferation of phagocytes, B cells and T cells (Glocker et al., Ann. NY Acad. Sci. 1246: 102-107, 2011; Moore et al., Annu. Rev. Immunol. 19: 683- 765 (2001); Waal Malefyt et al., J. Exp. Med. 174: 915-924, 1991), Williams et al., Immunol. 113: 281-292, 2004). In addition, it is a potent inhibitor of antigen presentation, inhibiting MHC class II expression as well as upregulation of costimulatory factors CD80 and CD86 (Mosser & Yhang, Immunol. Rev. 226: 205-218, 2008). Because of these characteristics, various studies have been conducted to use IL-10 as a therapeutic agent for inflammatory bowel disease or immune-related diseases such as psoriasis.

However, it is known that IL-10 has a dual characteristic that also has the opposite effect of immunostimulatory activity. In this regard, specifically, IL-10 stimulates B cell activation, prolongs the survival of B cells, and may contribute to class switching of B cells. It can also stimulate NK cell proliferation and cytokine production, and may act as a growth factor promoting the proliferation of a specific subset of CD8⁺ T cells (Mosser & Yhang, Immunol. Rev. 226: 205-218, 2009; Cai et al., Eur. J. Immunol. 29: 2658-2665, 1999; Santin et al., J. Virol. 74: 4729-4737, 2000; Rowbottom et al., Immunol. 160: 3188-3193, 1998). Importantly, it has been reported that high doses of IL-10 (20 and 25 µg/kg, respectively) in humans can increase the production of INFγ (Lauw et al., J. Immunol., 165: 2783-2789, 2000; Tilg et al., Gut 50: 191-195, 2002).

Accordingly, it has been reported that isoleucine, the 87^{th} amino acid of the IL-10 protein, is involved in immune activation, and when substituted with alanine, the immune activation may be suppressed (Ding et al., J. Exp. Med. 191(2): 213-223, 2000). However, in the case of the IL-10 variant protein, it has been confirmed that binding affinity with IL-10R1 is very weak compared to the wild type, and thus, it is disadvantageous in that a high dose of administration is required for equivalent immunosuppressive activity.

On the other hand, due to the structural properties of the IL-10 protein, a large amount of insoluble aggregates are generated when produced as a recombinant protein, which causes a great problem in productivity. Accordingly, a monomeric IL-10 that does not form a dimer has been developed by introducing a linker peptide of 6 a.a. between the fourth and fifth alpha helices based on the secondary structure of IL-10 (Josephson et al., J. Biol. Chem. 275(18): 13552-13557, 2000), but it has a short half-life and very low activity in the body, which is an obstacle to its use as a therapeutic agent. In order to overcome this low stability in the body, an attempt was made to express the IL-10 as a fusion protein linked to the Fc domain of IgA (Westerhof et al., PLOS ONE 7(10): e46460, 2012). But the fusion protein showed limited recovery of IL-10 activity.

### DISCLOSURE OF THE INVENTION

### TECHNICAL PROBLEM

The present invention is to solve various problems including the above-described problems, and the purpose of the present invention is providing a novel IL-10 variant protein which is more effective and whose *in vivo* stability and safety are enhanced. However, the scope of the present invention is not limited thereto.

### SUMMARY OF THE INVENTION

In an aspect of the present invention, there is provided a monomeric IL-10 variant protein in which a spacer peptide with a length of 6 to 12 a.a. are inserted between asparagine, the 116^{th} amino acid, and lysine, the 117^{th} amino acid, based on the mature form of the human Interleukin-10 (IL-10).

In another aspect of the present invention, there is provided a fusion protein comprising a monomeric IL-10 variant protein in which a spacer peptide with a length of 6 to 12 a.a. are inserted between asparagine, the 116^{th} amino acid, and lysine, the 117^{th} amino acid, based on the mature form of the human Interleukin-10 (IL-10).

In another aspect of the present invention, there is provided a polynucleotide encoding the monomeric IL-10 variant protein or the fusion protein.

In another aspect of the present invention, there is provided a recombinant vector comprising the polynucleotide.

In another aspect of the present invention, there is provided a pharmaceutical composition for immunosuppression comprising the monomeric IL-10 variant protein or the fusion protein as an active ingredient.

In another aspect of the present invention, there is provided a pharmaceutical composition for treating an immune-related disease comprising the monomeric IL-10 variant protein or the fusion protein as an active ingredient.

In another aspect of the present invention, there is provided a method for treating a subject suffering from an autoimmune disease comprising administering a therapeutically effective amount of the monomeric IL-10 variant protein or the fusion protein to the subject.

### EFFECT OF THE INVENTION

The monomeric IL-10 variant protein according to an embodiment of the present invention can be used as a novel immunosuppressant and/or therapeutic agent for treating autoimmune disease, inhibiting immune activation which is one of dual actions of IL-10 protein, improving production efficiency, as well as maintaining its activity in the form of fusion protein with other physiologically active proteins.

### BRIEF DESCRIPTION OF DRAWINGS

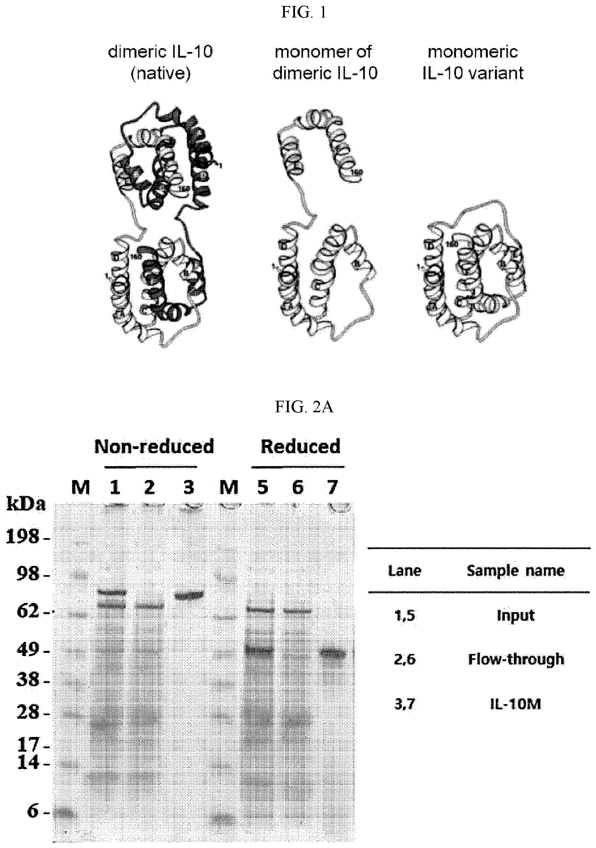

FIG. 1 is a structural diagram illustrating a three-dimensional structure of an IL-10 dimer (left), a monomer of the IL-10 dimer (center); and an IL-10 monomer having a stable monomer structure by inserting a spacer peptide according to an embodiment of the present invention (right).

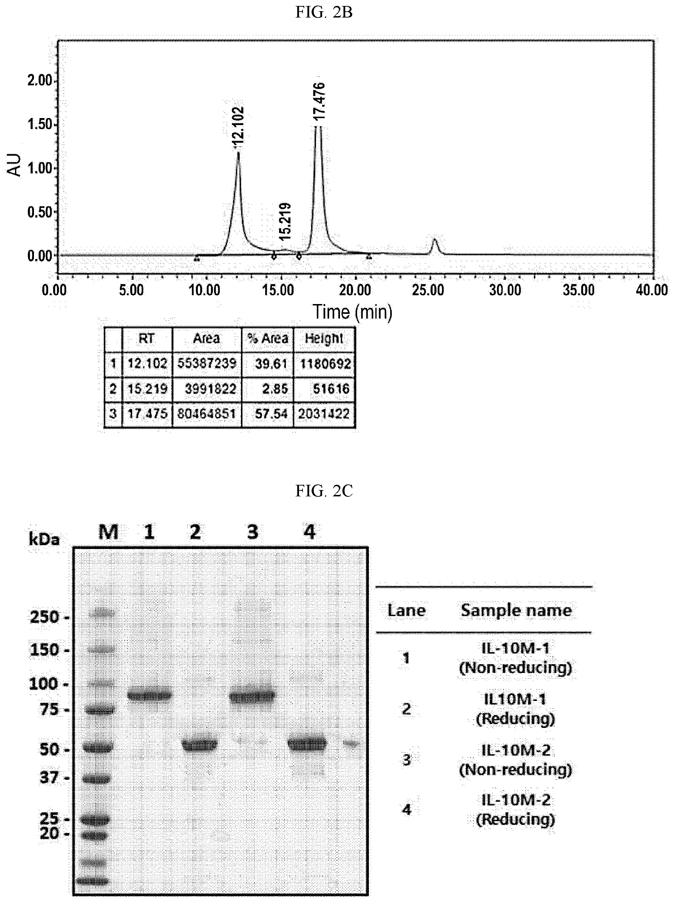

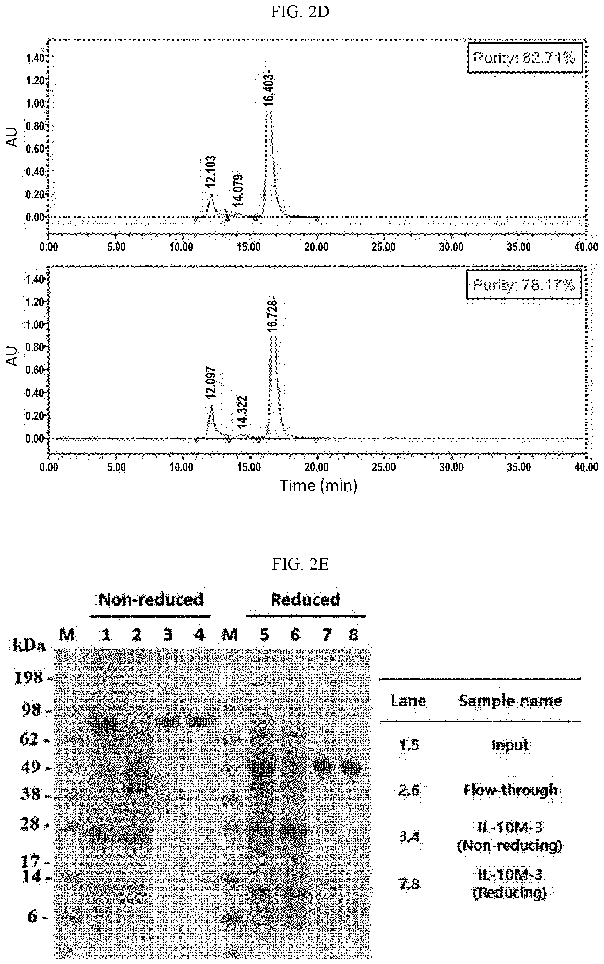

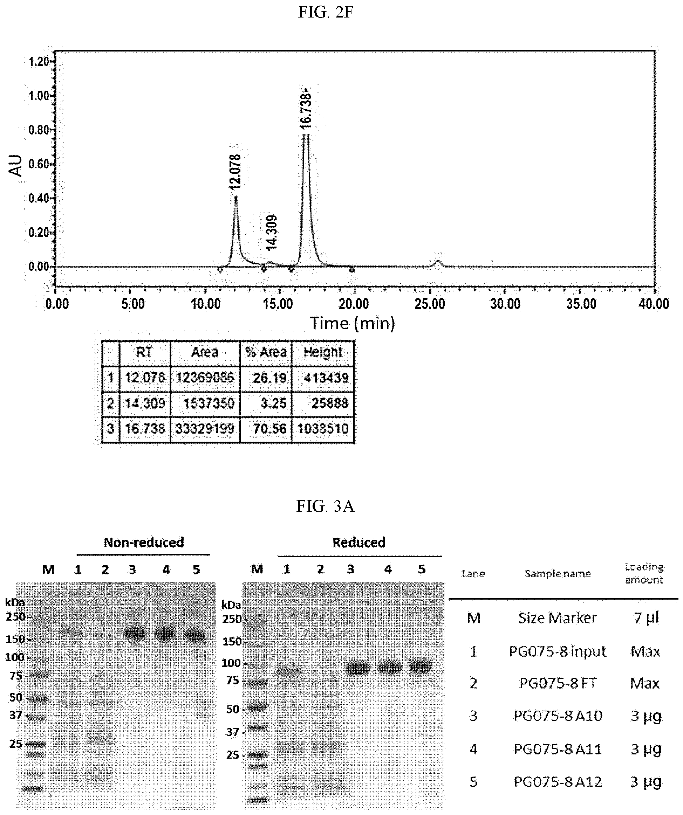

FIG. 2A is a photograph showing the result of SDS-PAGE analysis under non-reducing and reducing conditions of a fusion protein constructed by connecting a dimeric IL-10 variant protein to an Fc protein; and FIG. 2B is a chromatogram showing the results of analyzing the finally purified dimeric IL-10 variant fusion protein by SEC-HPLC analysis; FIG. 2C is a photograph showing the result of SDS-PAGE analysis in the purification procedure of the fusion proteins comprising a monomeric IL-10 variant protein according to examples 1 and 2 of the present invention linked to a Fc region; FIG. 2D is a series of chromatograms showing the purity of finally purified monomeric IL-10 variant fusion protein according to the Examples 1 (top) and 2 (bottom) of the present invention using SEC-HPLC analysis; and FIG. 2E is a photograph showing the result of SDS-PAGE analysis of the monomeric IL-10 variant fusion protein according to the Comparative example 2 of the present invention; and FIG. 2F is a chromatogram showing the purity of finally purified monomeric IL-10 variant fusion protein according to the Comparative example 2.

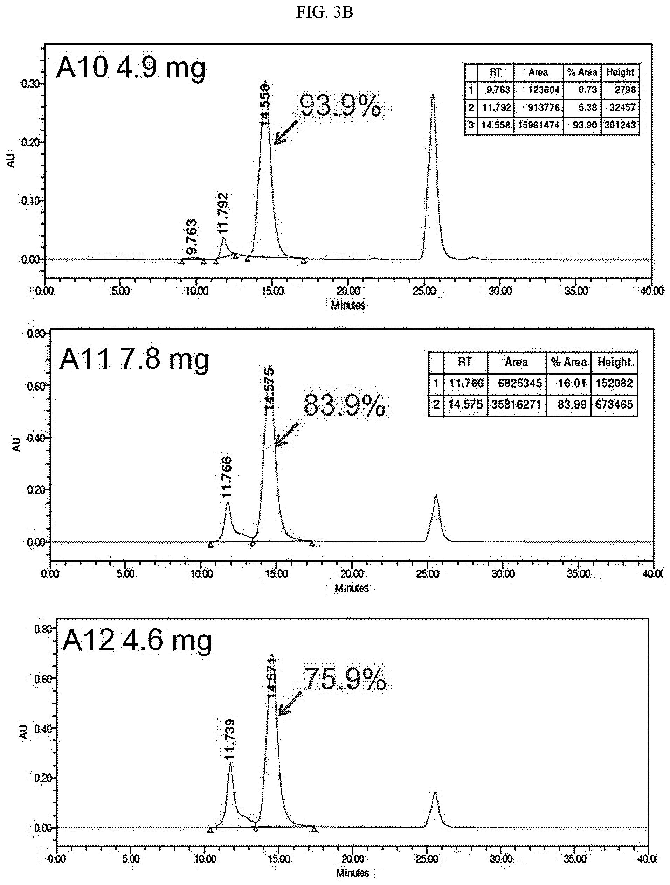

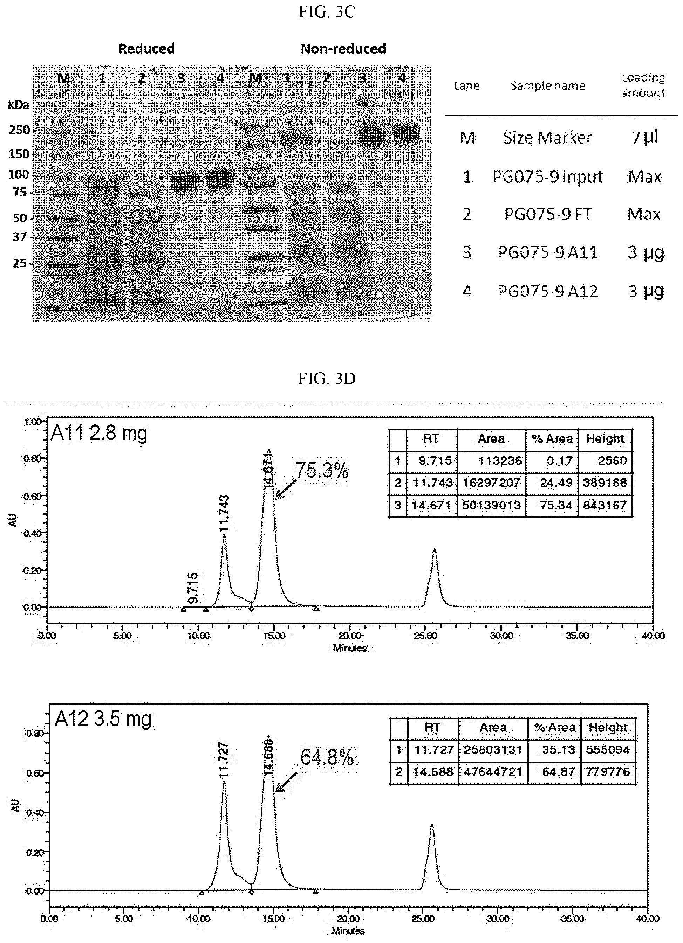

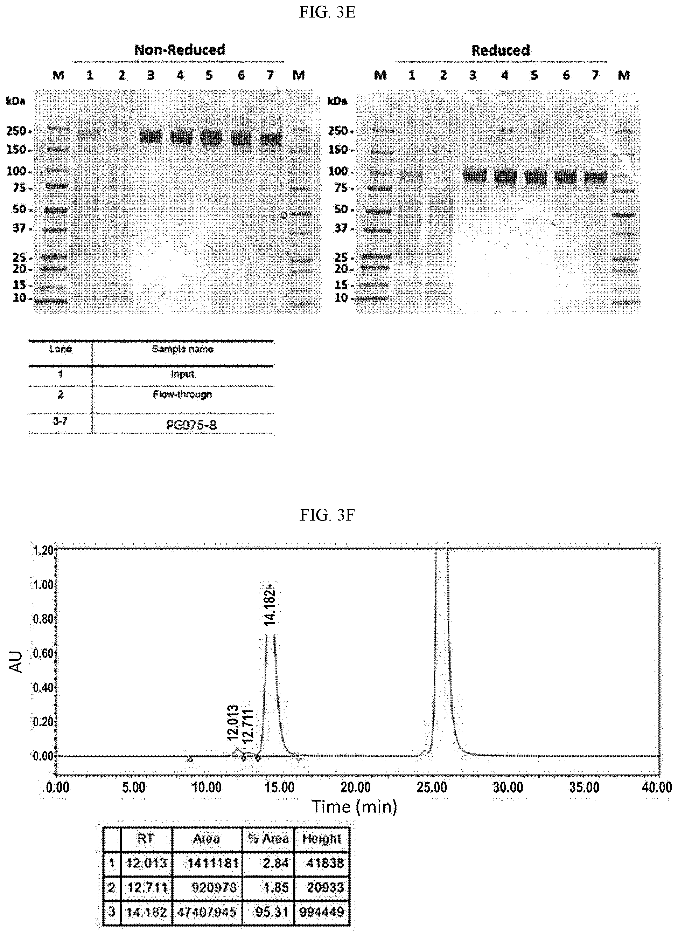

FIG. 3A is a photograph showing a result of SDS-PAGE analysis under a non-reducing condition (left) and a reduction condition (right) during purifying the fusion protein (PG075-8) according to the Example 3 of the present invention using protein A after transiently expressing the fusion protein in transfected cells; and FIG. 3B is a chromatogram showing the result of analyzing purity of the fusion protein according to the present invention in the 10^{th} fraction (A10) to 12^{th} fraction (A12) during purification from transiently transfected cells; FIG. 3C is a photograph showing the result of SDS-PAGE analysis under non-reducing condition (right) and reducing condition (left) in the purification procedure of the fusion protein of the present invention using protein A after expressing in transiently transfected cells; FIG. 3D is a chromatogram showing the result of analyzing purity of the fusion protein (PG075-9) according to the present invention in the 11^{th} fraction (A11) and 12^{th} fraction (A12) during purification from transiently transfected cells; FIG. 3E is a photograph showing the result of SDS-PAGE analysis under non-reduction condition (left) and reduction condition (right) to check the degree of expression of the fusion protein (PG075-8) according to the Example 3 of the present invention expressed from the stable cell line proteins prepared for producing the fusion protein according to the purification procedures; and FIG. 3F is a chromatogram showing the result of SEC-HPLC analysis to check the purity of the finally purified fusion protein (PG075-8) in the stable cell line.

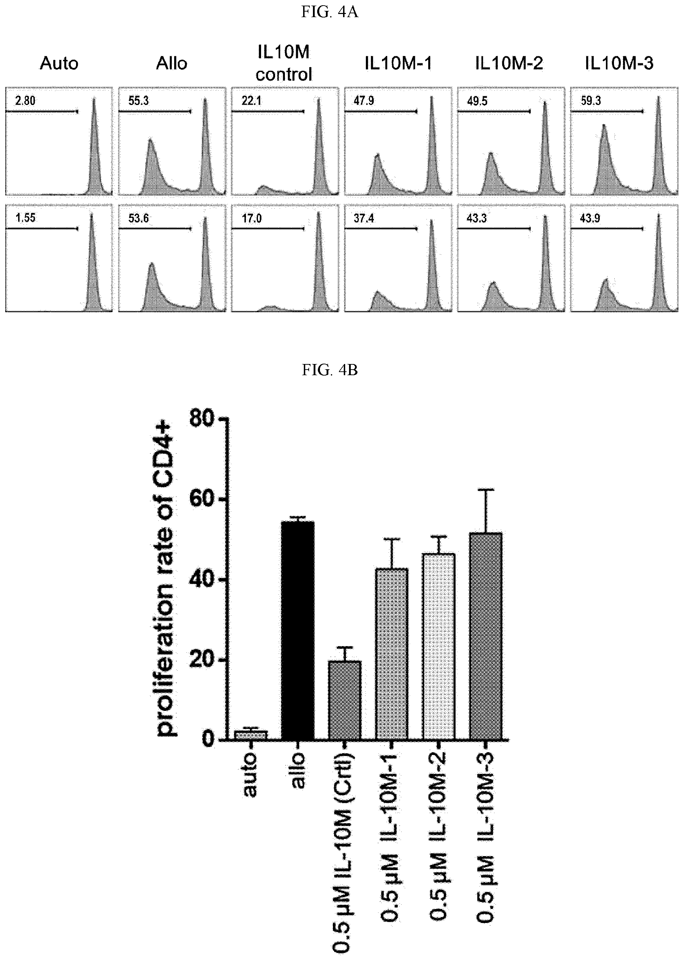

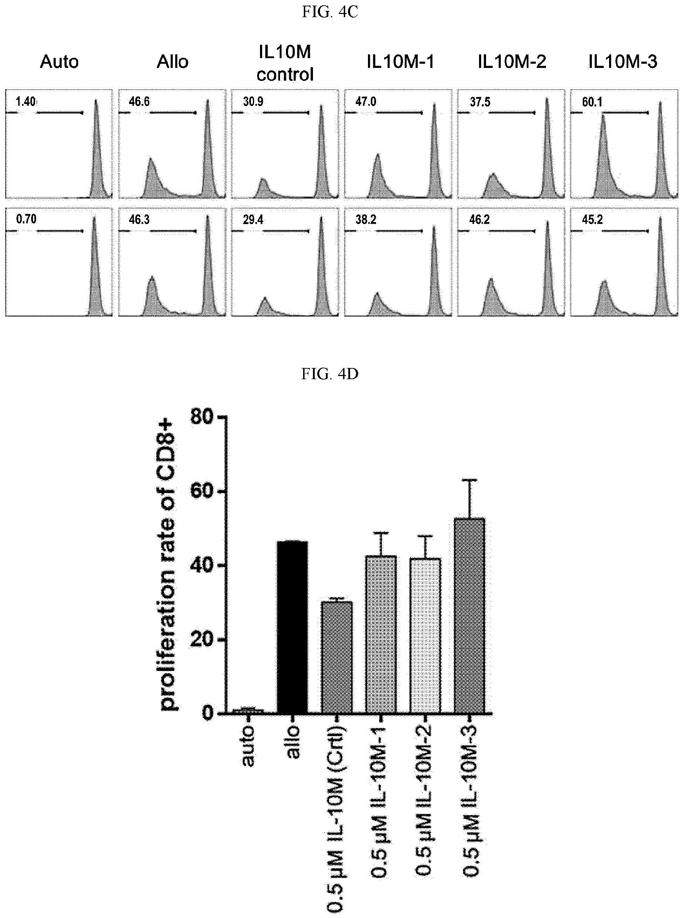

FIG. 4A is a series of histograms showing the results of investigating the effects of the IL-10 fusion proteins according to the Comparative Examples 1 and 2 and Examples 1 and 2 of the present invention on the proliferation of CD4⁺ T cells using FACS analysis; FIG. 4B is a graph quantifying the results of FIG. 4A; FIG. 4C is a histogram showing the results of investigating the effects of the IL-10 fusion proteins according to the Comparative Examples 1 and 2 and Examples 1 and 2 of the present invention on the proliferation of CD4⁺ T cells using FACS analysis; and FIG. 4D is a graph quantifying the result of FIG. 4C.

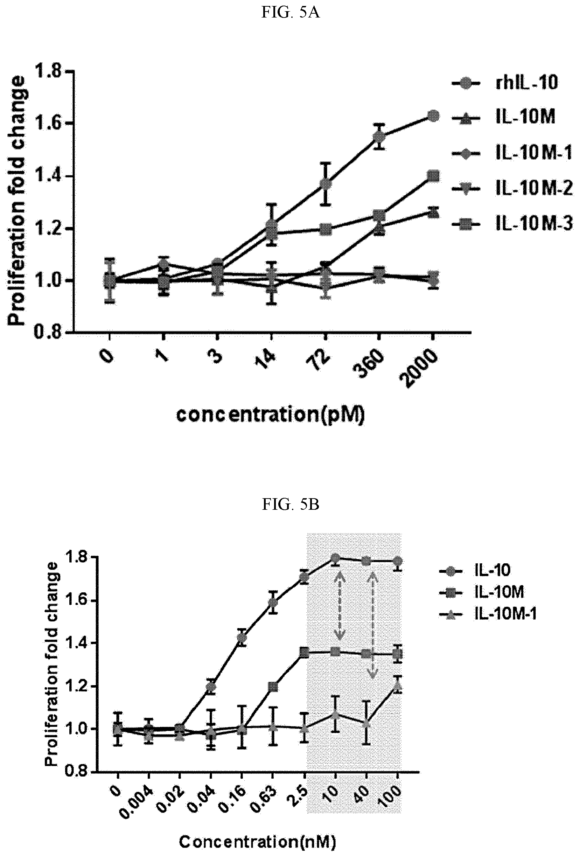

FIG. 5A is a graph showing the results of analyzing the effect of rhIL-10 (control) and IL-10 variant fusion proteins according to the Comparative Examples 1 and 2 and Examples 1 and 2 of the present invention on the proliferation of bone marrow-derived mast cells depending on treated concentration; and FIG. 5B is a graph showing the results of analyzing the effect of rhIL-10 (control) and IL-10 variant fusion proteins according to the Comparative Example 1 and Example 1 of the present invention on the proliferation of bone marrow-derived mast cells after increasing treatment concentration of the variant fusion proteins.

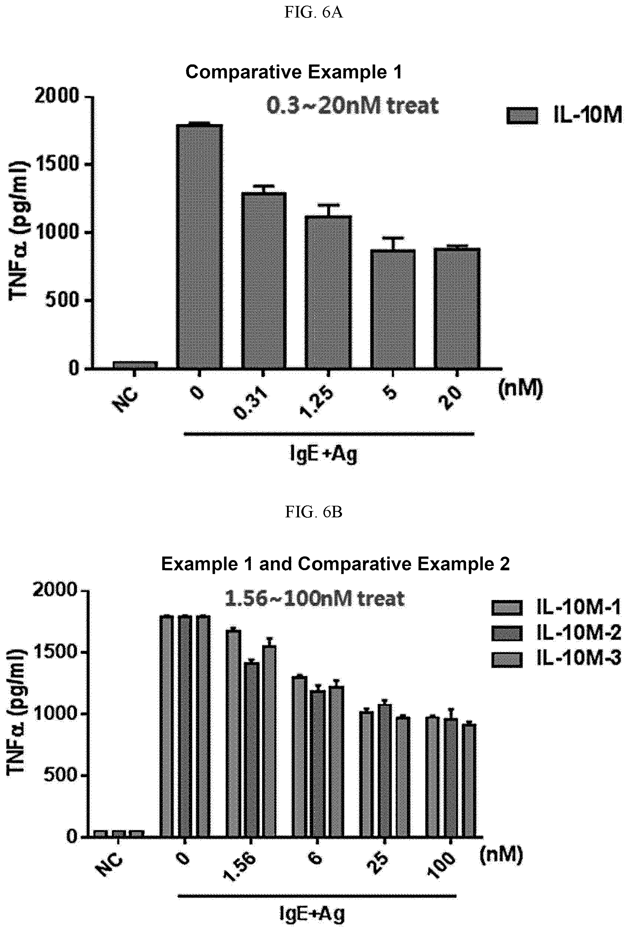

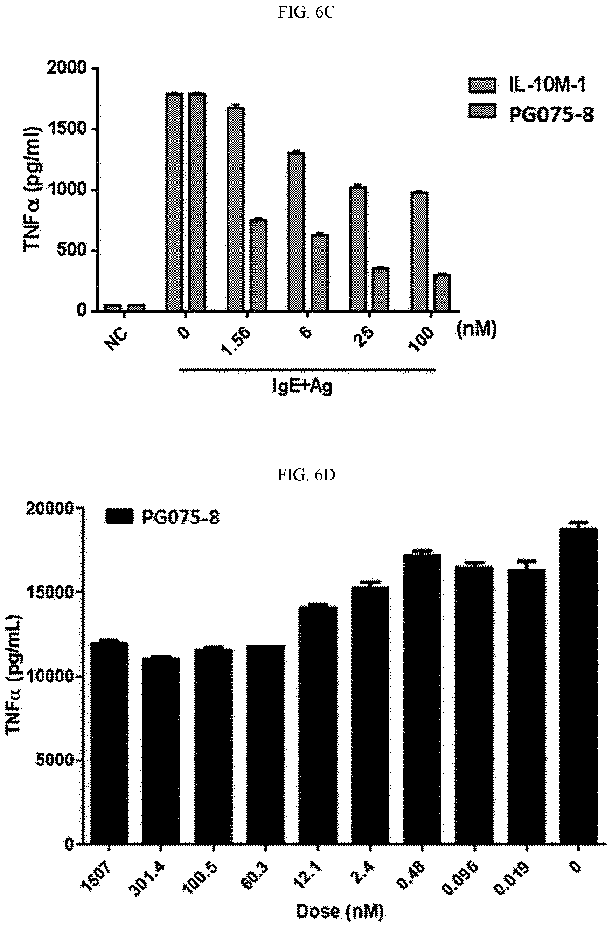

FIG. 6A is a graph showing the results of analyzing the change in the TNF-α secretion in mast cells treated with the dimeric IL-10 variant fusion protein according to the Comparative Example 1 depending on treated concentration; FIG. 6B is a graph showing the results of analyzing the change in the TNF-α secretion in mast cells treated with the monomeric IL-10 variant fusion proteins according to the Comparative Examples 2 and Examples 1 and 2 of the present invention depending on treated concentration; FIG. 6C is a graph showing the results of analyzing TNF-α secretion-inhibitory activity of the fusion protein PG075-8 according an embodiment of the present invention and dimeric IL-10 variant proteins (IL-10M-1; Fc-IL-10Vm) as controls in mast cells; and FIG. 6D is a graph showing the results of analyzing TNF-α secretion-inhibitory activity of the fusion protein PG075-8 according to an embodiment of the present invention in macrophages depending on treated concentration.

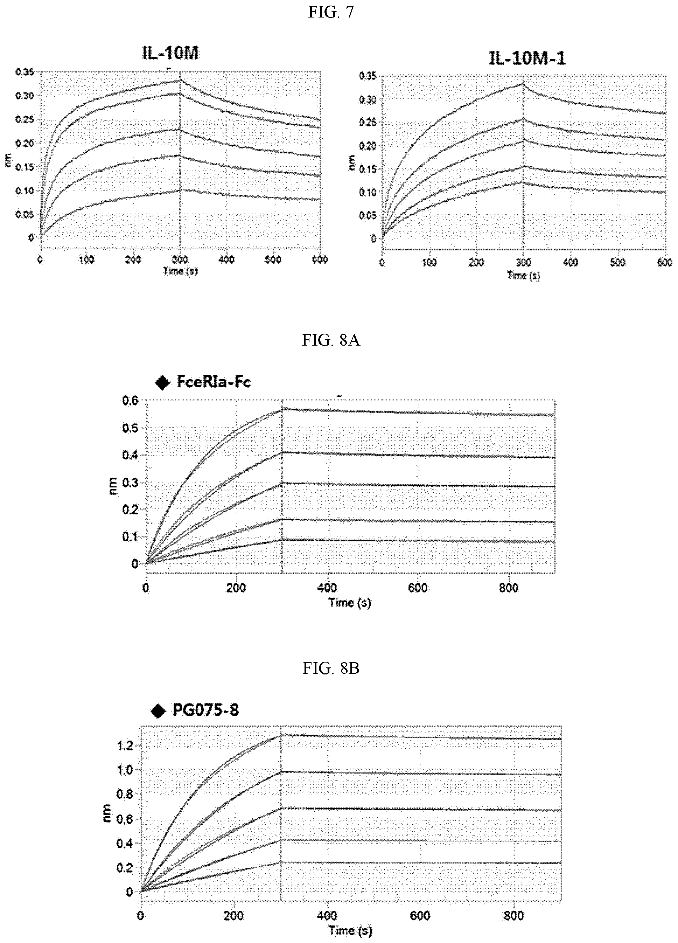

FIG. 7 is a series of sensograms showing the results of analysis of binding affinity of the dimeric IL-10 variant fusion protein according to the Comparative Example 1 (left) and the monomeric IL-10 variant fusion protein according to the Example 1 (right) of the present invention to IL-10R1 depending on treated concentration through BLI analysis.

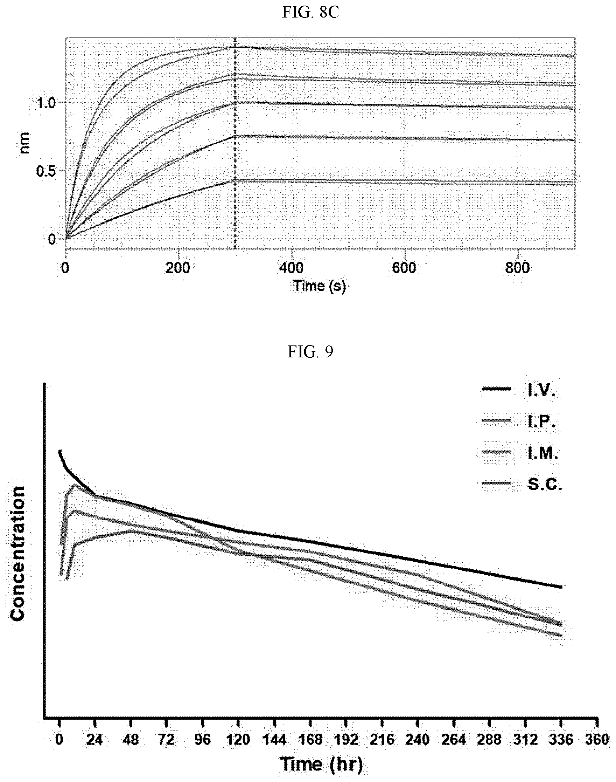

FIG. 8A is a senosogram showing the result of analysis of binding affinity of FcεRIα-Fc as a control to mouse IgE through BLI analysis; and FIG. 8B is an sensogram showing the result of analysis of binding affinity of the fusion protein PG075-8 (FcεRIα-Fc-IL-10Vm; bottom) according to the Example 3 of the present invention to mouse IgE through BLI analysis; and FIG. 8C is a sensogram showing the result of analysis of binding affinity of the fusion protein PG075-8 (FcεRIα-Fc-IL-10Vm) according to the Example 3 of the present invention to human IgE through BLI analysis.
FIG. 9 is a graph illustrating a result of pharmacokinetics analysis by quantifying the amount of fusion proteins remaining in the serum for up to 330 hours after administering the fusion proteins according to embodiments of the present invention to experimental animals (rat) through various routes (intravenous, intraperitoneal, intramuscular, and subcutaneous injection).

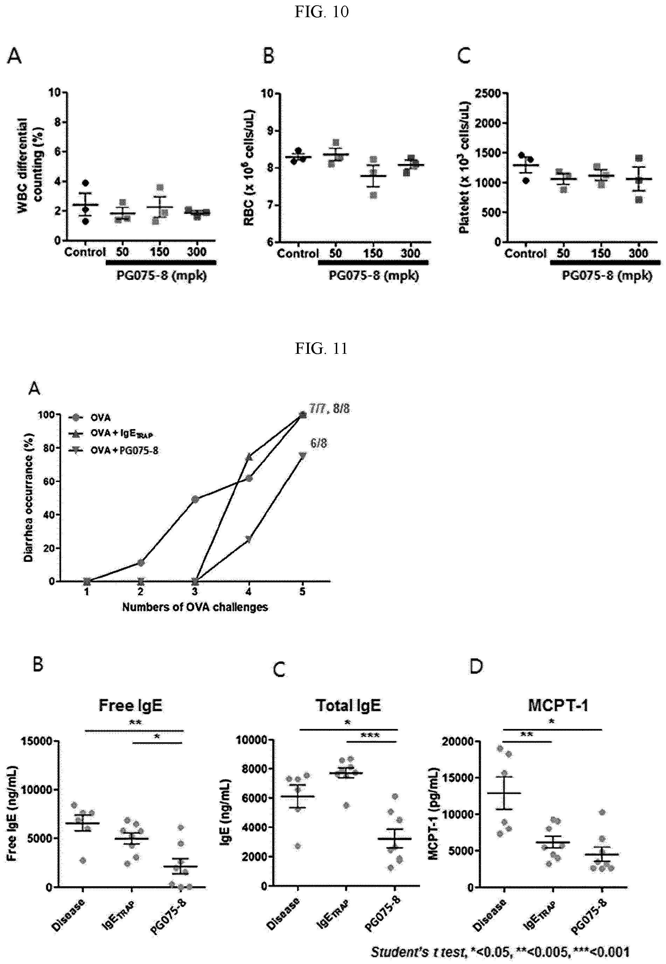

FIG. 10 is a series of graphs showing the results of hemotoxicity analysis examining whether the number of white blood cells (A), red blood cells (B), and platelets (C) changed when the fusion protein PG075-8 according to an embodiment of the present invention is administered to experimental animals.
FIG. 11 relates to the result of the investigation whether the fusion protein PG075-8 according to an embodiment of the present invention reduces allergic symptoms in experimental animals. FIG. 11 shows a series of graphs representing the results of analysis of (A) change of symptom of diarrhea when PG075-8 according to an embodiment of the present invention and IgE TRAP (control) were administrated, respectively after inducing food allergy via oral administration of OVA to OVA-sensitized mice; (B) concentration of free IgE determined from sacrificed mice after completion of experiment; (C) concentration of total IgE a an allergy indicator; and (D) concentration of degranulating enzyme (mast cell protease-1, MCPT-1) in blood mast cells as an allergy indicator.

### BEST MODES OF THE INVENTION

In an aspect of the present invention, there is provided a monomeric IL-10 variant protein in which a spacer peptide with a length of 6 to 12 a.a. are inserted between asparagine, the 116^{th} amino acid, and lysine, the 117^{th} amino acid, based on the mature form of the human Interleukin-10 (IL-10).

In the monomeric IL-10 variant protein, the mature form of the human Interleukin-10 may be one derived from 19^{th} to 178^{th} amino acid sequence described in UniProtKB P22301.

In monomeric IL-10 variant protein, the spacer peptide may have 7 to 11 a.a., 8 to 10 a,a., or 9 a.a. of length, or alternatively the space peptide may have length of 6, 7, 8, 9, 10, 11 or 12 a.a. In a particular embodiment, the spacer peptide may be a peptide having amino acid sequence of GGSGGSGGS(SEQ ID NO: 4), (GGGSGG)ₙ (unit: SEQ ID NO: 5, n is an integer of 1 or 2), (G₄S)n (unit: SEQ ID NO: 12, n is an integer of 1 or 2), (GGS)ₙ (n is an integer of 2 to 4), (GS)ₙ (n is an integer of 3 to 6), or (GSSGGS)n (unit: SEQ ID NO: 13, n is an integer of 1 to 2). The amino acid residues consisting of the spacer peptide may be substituted with other type of amino acids as long as they do not induce any adverse immunogenic reactions and preferably may have length of 9 a.a.

The term "spacer peptide" used herein refers to a peptide that is inserted into a specific protein and plays a role in changing the structure and/or function of the protein. In this sense, it is distinguished from linker peptides connecting other fusion partners, but conventional linker peptides can be used as spacer peptides.

The monomeric IL-10 variant protein according to an embodiment of the present invention may include an amino acid sequence of SEQ ID NO: 1.

The monomeric IL-10 variant protein may be a monomeric IL-10 variant protein in which isoleucine, the 87^{th} amino acid, is substituted with alanine based on the mature form of the human IL-10 protein, and preferably may have an amino acid sequence of SEQ ID NO: 39.

The monomeric IL-10 variant protein may have homology of 90%, 91%, 92%, 93%, 94%, 95%, 96%, 97%, 98%, or 99% with the amino acid sequence represented by SEQ ID NOs: 1 or 39 as long as it has a monomeric structure, and it may be derived from mammals.

In another aspect of the present invention, there is provided a fusion protein comprising a monomeric IL-10 variant protein in which a spacer peptide with a length of 6 to 12 a.a. are inserted between asparagine, the 116^{th} amino acid, and lysine, the 117^{th} amino acid, based on the mature form of the human Interleukin-10 (IL-10).

As used herein, the term "fusion protein" refers to a recombinant protein in which two or more proteins or domains responsible for specific functions in a protein are linked such that each protein or domain is responsible for an original function thereof. A linker peptide having a flexible structure can be generally inserted between the two or more proteins or domains. The linker peptide may have any one among following amino acid sequences: AAGSGGGGGSGGGGSGGGGS (SEQ ID NO: 2), GGSGG (SEQ ID NO: 3), GGSGGSGGS (SEQ ID NO: 4), GGGSGG (SEQ ID NO: 5), (G₄S)ₙ (unit: SEQ ID NO: 12, n is an integer of 1 to 10), (GGS)ₙ (n is an integer of 1 to 10), (GS)ₙ (n is an integer of 1 to 10), (GSSGGS)ₙ (unit: SEQ ID NO: 13, n is an integer of 1 to 10), KESGSVSSEQLAQFRSLD (SEQ ID NO: 14), EGKSSGSGSESKST (SEQ ID NO: 15), GSAGSAAGSGEF (SEQ ID NO: 16), (EAAAK)n (unit: SEQ ID NO: 17, n is an integer of 1 to 10), CRRRRRREAEAC (SEQ ID NO: 18), A(EAAAK)₄ALEA(EAAAK)₄A (SEQ ID NO: 19), GGGGGGGG (SEQ ID NO: 20), GGGGGG (SEQ ID NO: 21), AEAAAKEAAAAKA (SEQ ID NO: 22), PAPAP (SEQ ID NO: 23), (Ala-Pro)n (n is an integer of 1 to 10), VSQTSKLTRAETVFPDV (SEQ ID NO: 24), PLGLWA (SEQ ID NO: 25), TRHRQPRGWE (SEQ ID NO: 26), AGNRVRRSVG (SEQ ID NO: 27), RRRRRRRR (SEQ ID NO: 28), GFLG (SEQ ID NO: 29), GSSGGSGSSGGSGGGDEADGSRGSQKAGVDE (SEQ ID NO: 30), GGGGSGGGGSGGGGSEPKSSDKTHTCPPCP (SEQ ID NO: 31), GGGGSGGGGSGGGGS (SEQ ID NO: 34), GGGGSGGGGSGGGGSEKEKEEQEERTHTCPPCP (SEQ ID NO: 35), RNTGRGGEEKKGSKEKEEQEERETKTPECP (SEQ ID NO: 36), GGGGSGGGGSGGGGSEPKSCDKTHTCPPCP (SEQ ID NO: 37), GSGGGSGTLVTVSSESKYGPPCPPCP (SEQ ID NO: 38), EPKSSDKTHTCPPCP (SEQ ID NO: 40), EPKSCDKTHTCPPCP (SEQ ID NO: 41), THTCPPCP (SEQ ID NO: 42), GGGGSGGGGSGGGGSAKNTTAPATTRNTTRGGEEKKKEKEKEEQEERTHTCPPCP (SEQ ID NO: 43), AGSGGGGGSGGGGSGGGGS (SEQ ID NO: 44), and GGGSGGSTHTCPPCP(SEQ ID NO: 45).

The fusion protein may include one or more fusion partner proteins that perform different functions. Such fusion partner proteins may be an antibody specifically binding to a target protein, an antigen-binding fragment of the antibody, an antibody mimetic specifically binding to the target protein, an antibody Fc region, an antibody Fc region receptor, or an extracellular domain of the antibody Fc region receptor, a dimerization domain, a cytokine or an immunomodulatory peptide.

In the fusion protein, the antigen-binding fragment of the antibody may be Fab, F(ab')₂, Fab', scFv, diabody, triabody, sdAb (single domain antibody), V_{NAR} or V_{H}H, and the antibody mimetic may be affibody, affilin, affimer, affitin, alphabody, anticalin, avimer, DARPin, Fynomer, Kunitz domain peptide, monobody, repebody, VLR, or nanoCLAMP The Fc region may an Fc region of IgG, IgA, IgD, IgE, IgM or the Fc region may be a hybrid Fc (hyFc) in which two or more domains (hinge, CH2, and CH3 domain) of Fc regions of the above-described Ig subclasses are mixed, and the IgG may be IgG1, IgG2, IgG3, or IgG4. More specifically, the Fc region may be a variant Fc region whose functional parts (effectors) responsible for antibody-dependent cell-mediated cytotoxicity (ADCC) or complement-dependent cytotoxicity (CDC) are mutated in order to lower affinity for Fc gamma receptor (FcyRc) and a complement (C1q) and/or a variant Fc region engineered to improve selective affinity to neonatal Fc receptor (FcRn) having elevated blood half-life thereby. Among these, the hybrid Fc may be those described in Korean Patent Nos. 897938, 1380732, and 1380729, etc., or the variant Fc region may be a modified immunoglobulin Fc protein (NTIG) described in International Patent Application PCT/KR2020/006346. More specifically, the Fc region may contain an amino acid sequence selected from the group consisting of SEQ ID NOs: 6 to 9. The term 'NTIG' used herein refers to a modified Fc domain protein in which 18^{th} and 196^{th} amino acids of the hybrid Fc protein including an amino acid sequence selected from the group consisting of SEQ ID NOs: 6 and 7 are mutated to other amino acids, that lacks effector functions such as ADCC and CDC, but has improved blood half-life by increasing selective affinity for FcRn. The NTIG may contain an amino acid sequence selected from the group consisting of SEQ ID NOs: 8 and 9.

The dimerization domain may be a hinge domain of antibody, LIM/double zinc-finger motif, RAG1 domain, HAT dimerization domain, TRFH dimerization domain, Stat3 dimerization domain, or LFB1/HNF1 dimerization domain, but not limited thereto. The cytokine may be IL-4, IL-6, IL-1α or TGF-β. The immunomodulatory peptide may be PD-1L or CTLA-4 (CD 152). In particular, if an Fc domain is a fusion partner protein, a dimer may be formed by intermolecular disulfide bonds generated between cysteine groups present in the hinge region. The fusion partner protein may be linked to either the N-terminus or the C-terminus of the monomeric IL-10 variant protein according to an embodiment of the present invention. In addition, in this case, the fusion partner protein may be linked to the monomeric IL-10 variant protein through various linker peptides described above.

As used herein, the term "antibody" refers to an immunoglobulin molecule which is a hetero-tetrameric protein produced by binding two identical heavy chains and two identical light chains, and performs antigen-specific binding through an antigen-binding site composed of a variable region (V_{L}) of the light chain and a variable region (V_{H}) of the heavy chain, thereby causing an antigen-specific humoral immune response.

As used herein, the term "antigen-binding fragment of an antibody" refers to a fragment which has antigen-binding ability derived from an antibody and includes both a fragment produced by cleaving an antibody with a protein cleaving enzyme as well as a single-chain fragment produced in a recombinant manner, and examples thereof include Fab, F(ab')₂, scFv, diabody, triabody, sdAb, and V_{H}H.

As used herein, the term "Fab" refers to an antigen-binding antibody fragment (fragment antigen-binding) which is produced by cleaving an antibody molecule with a proteolytic enzyme, papain, is a heterodimer of two peptides of V_{H}-CH1 and V_{L}-C_{L}, and the other fragment produced by the papain is referred to as Fc (fragment crystallizable).

As used herein, the term "F(ab')₂" refers to a fragment that includes an antigen-binding site among fragments produced by cleaving an antibody with pepsin, which is a proteinase, and is in a form of a tetramer in which the two Fab's are linked by a disulfide bond. The other fragment produced by the pepsin is referred to as pFc'.

As used herein, the term "Fab" refers to a molecule having a similar structure to that of Fab produced by separating the abovementioned F(ab')₂under weak reducing conditions.

As used herein, the term "scFv" is an abbreviation for a "single chain variable fragment", and refers to a fragment which is not a fragment of an actual antibody, but is a kind of fusion protein prepared by linking the heavy-chain variable region (V_{H}) to the light-chain variable region (V_{L}) of the antibody through a linker peptide having a size of about 25 a.a., and is known to have antigen-binding ability even though the fragment is not a unique antibody fragment (Glockshuber et al., Biochem. 29(6): 1362-1367, 1990).

As used herein, the terms "diabody" and "triabody" refer to antibody fragments in a form of two and three scFv's linked by a linker, respectively.

As used herein, the term "single domain antibody (sdAb)" refers to an antibody fragment which is also referred to as a nanobody and consists of a single variable region fragment of an antibody. The sdAb derived from the heavy chain is mainly used, but a single variable region fragment derived from the light chain is also reported to specifically bind to an antigen. V_{NAR} composed of variable region fragments of a shark antibody and V_{H}H composed of variable region fragments of a camelid antibody, which consist only of dimers of single chains unlike conventional antibodies composed of a heavy chain and a light chain, are also included in sdAb.

As used herein, the term "antibody mimetic" or alternatively "antibody analog" is a concept including a protein having similar functions to those of antibodies prepared from non-antibody-derived protein scaffolds such as a monobody and a variable lymphocyte receptor (VLR), that is, having antigen-binding ability, unlike a normal full-length antibody in which two heavy chains and two light chains form a quaternary structure of a hetero-tetramer to exhibit functions. Examples of such an antibody mimetic include Affibody derived from a Z domain of protein A (Nygren, P. A., FEBS J. 275(11): 2668 to 2676, 2008), Affilin derived from Gamma-B crystallin or Ubiquitin (Ebersbach et al., J. Mol. Biol. 372(1): 172-185, 2007), Affimer derived from Cystatin (Johnson et al., Anal. Chem. 84(15): 6553-6560, 2012), Affitin derived from Sac7d (Krehenbrink et al., J. Mol. Biol. 383 (5): 1058-1068, 2008), Alphabody derived from a triple helix coiled coil protein (Desmet et al., Nat. Commun. 5: 5237, 2014), Anticalin derived from lipocalin (Skerra et al., FEBS J. 275(11): 2677-2683, 2008), Avimer derived from domains of various membrane receptors (Silverman et al., Nat. Biotechnol. 23(12): 1556-1561, 2005), DARPin derived from Ankyrin repeat motif (Stumpp et al., DrugDiscov. Today. 13(15-16): 695-701, 2008), Fynomer derived from a SH3 domain of a Fyn protein (Grabulovski et al., J. Biol. Chem. 282(5): 3196-3204, 2007), Kunitz domain peptides derived from Kunitz domains of various protein inhibitors (Nixon and Wood, Curr. Opin. Drug Discov. Dev. 9(2): 261-268, 2006), a monobody derived from the 10^{th} type 3 domain of fibronectin (Koide and Koide, Methods Mol. Biol. 352: 95-109, 2007), nanoCLAMP derived from carbohydrate-binding module 32-2 (Suderman et al., Protein Exp. Purif. 134: 114-124, 2017), a variable lymphocyte receptor (VLR) derived from a hagfish (Boehm et al., Ann. Rev. Immunol. 30: 203-220, 2012), and a repebody engineered to enhance antigen affinity based on the VLR (Lee et al., Proc. Natl. Acad. Sci. USA, 109: 3299-3304, 2012).

In the fusion protein, the antibody Fc region receptor may be an extracellular domain of alpha subunit of IgE Fc receptor.

In another aspect of the present invention, there is provided a polynucleotide encoding the monomeric IL-10 variant protein or the fusion protein.

In another aspect of the present invention, there is provided a recombinant vector comprising the polynucleotide.

In the recombinant vector, the polynucleotide may be contained in a form of a gene construct operably linked to a regulatory sequence.

As used herein, the term "operably linked to" means that a target nucleic acid sequence (for example, *in vitro* transcription/translation system or in a host cell) is linked to the regulatory sequence in such a way that the target nucleic acid sequence can be expressed.

As used herein, the term "regulatory sequence" is meant to include a promoter, an enhancer, and other regulatory elements (for example, polyadenylation signal). Examples of the regulatory sequence include a sequence which directs such that a target nucleic acid is constantly expressed in many host cells, a sequence (for example, a tissue-specific regulatory sequence) which directs such that a target nucleic acid is expressed only in a specific tissue cell, and a sequence (for example, an inducible regulatory sequence) which directs such that expression is induced by a specific signal. Those skilled in the art could understand that the design of an expression vector may vary depending on factors such as the selection of a host cell to be transformed and the desired level of protein expression. The expression vector of the present invention can be introduced into a host cell to express the fusion protein. Regulatory sequences which enable expression in the eukaryotic cell and the prokaryotic cell are well known to those skilled in the art. As described above, these regulatory sequences generally include regulatory sequences responsible for transcription initiation, and optionally, a poly-A signal responsible for transcription termination and stabilization of a transcript. Additional regulatory sequences may include a translation enhancing factor and/or a naturally-combined or heterologous promoter region, in addition to the transcription regulatory factor. For example, possible regulatory sequences which enable expression in a mammalian host cell include a CMV-HSV thymidine kinase promoter, SV40, an RSV (Rous sarcoma virus)-promoter, a human kidney urea 1α-promoter, a glucocorticoid-inducing MMTV (Moloney mouse tumor virus)-promoter, a metallothionein- or tetracycline-inducible promoter, or an amplifying agent such as a CMV amplifying agent and an SV40 amplifying agent. It is considered that for expression in a nerve cell, a neurofilament-promoter, a PGDF-promoter, an NSE-promoter, a PrP-promoter, or a thy-1-promoter can be used. The abovementioned promoters are known in the art, and are described in the literature (Charron, J. Biol. Chem. 270: 25739 to 25745, 1995). For the expression in the prokaryotic cell, a number of promoters, including a lac-promoter, a tac-promoter, or a trp promoter, have been disclosed. In addition to the factors capable of initiating transcription, the regulatory sequences may include a transcription termination signal, such as an SV40-poly-A site and a TK-poly-A site, on the downstream of the polynucleotide according to one exemplary embodiment of the present invention. In the present specification, suitable expression vectors are known in the art, and examples thereof include Okayama-Berg cDNA expression vector pcDV1 (Parmacia), pRc/CMV, pcDNA1, pcDNA3 (Invitrogen), pSPORT1 (GIBCO BRL), pGX-27 (Korean Patent No. 1442254), pX (Pagano et al., Science 255: 1144-1147, 1992), a yeast two-hybrid vector such as pEG202 and dpJG4-5 (Gyuris et al., Cell 75: 791-803, 1995), and a prokaryotic expression vector such as lambda gt11 and pGEX (Amersham Pharmacia). The vector may further include a polynucleotide encoding a secretion signal, in addition to the nucleic acid molecules of the present invention. The secretion signals are well known to those skilled in the art. Moreover, depending on the used expression system, a leader sequence which can lead the fusion protein according to one exemplary embodiment of the present invention to a cellular compartment is combined with a coding sequence of the polynucleotide according to one exemplary embodiment of the present invention, and is preferably a leader sequence capable of directly secreting a decoded protein or the protein thereof into a pericytoplasmic or extracellular medium.

In addition, the vector of the present invention can be prepared, for example, by a standard recombinant DNA technique, and examples of the standard recombinant DNA technique include ligation of a smooth terminus and an adhesion terminus, a restriction enzyme treatment to provide a proper terminus, removal of a phosphate group by an alkaline phosphatase treatment to prevent inappropriate binding, and enzymatic linkage by T4 DNA ligase. The vector of the present invention can be prepared by recombining DNA encoding a signal peptide obtained by chemical synthesis or a genetic recombination technique, the immunoglobulin Fc domain variant protein according to one exemplary embodiment of the present invention, or DNA encoding a fusion protein containing the same with a vector containing an appropriate regulatory sequence. The vector containing a regulatory sequence can be commercially purchased or prepared, and in one exemplary embodiment of the present invention, a pBispecific backbone vector (Genexine, Inc., Korea), a pAD15 vector, pGP30 (Genexine, Inc. Korea), or a pN293F vector (Y-Biologics, Inc., Korea) was used as a backbone vector.

The expression vector may further include a polynucleotide encoding a secretion signal sequence, and the secretion signal sequence induces the extracellular secretion of the recombinant protein expressed in the cell, and may be a tissue plasminogen activator (tPA) signal sequence, a herpes simplex virus glycoprotein Ds (HSV gDs) signal sequence, or a growth hormone signal sequence.

The expression vector according to one exemplary embodiment of the present invention may be an expression vector capable of expressing the protein in a host cell, and the expression vector may be in any form such as a plasmid vector, a viral vector, a cosmid vector, a phagemid vector, or an artificial human chromosome.

In another aspect of the present invention, there is provided a pharmaceutical composition for immunosuppression comprising the monomeric IL-10 variant protein or the fusion protein as an active ingredient.

The pharmaceutical composition may further contain a known immunosuppressant component (a cytokine with immunosuppressive effects, a Decoy receptor, a ligand involved in the activation and differentiation of immune cells and an antibody against the ligand, and an antibody capable of inhibiting immune cell activity, etc.). These known immunosuppressants include a glucocorticoid, a cytostatic agent, an anti-CD20 antibody, an anti-CD3 antibody, an anti-IL-2 antibody, an immunophilin inhibitor, interferon β, opioid, TNFα binding protein, mycophenolate, fingolimod or myriocin. The glucocorticoid may be prednisone, dexamethasone, or hydrocortisone. The cytostatic agent may be nitrogen mustard, nitrosourea, platinum coordination complex, folic acid analogue, azathioprine, mercaptopurine, fluorouracil, methotrexate, dactinomycin, anthracycline, mitomycin C, bleomycin or mithramycin. The immunophilin inhibitor may be cyclosporin, tacrolimus, sirolimus, or everolimus.

According to another aspect of the present invention, there is provided a pharmaceutical composition for the treatment of an immune-related disease comprising the monomeric IL-10 variant protein or fusion protein as an active ingredient.

In the pharmaceutical composition, the immune-related disease may be type 1 diabetes, alopecia areata, anti-phospholipid antibody syndrome, rheumatoid arthritis, psoriasis or psoriatic arthritis, multiple sclerosis, systemic lupus erythematosus, inflammatory bowel disease, Addison's disease, Graves' disease, Sjögren's syndrome, Guillain-Barre syndrome, Hashimoto's thyroiditis, Myasthenia gravis, inflammatory myopathy, autoimmune vasculitis, autoimmune hepatitis, hemorrhagic anemia, idiopathic thrombocytopenic purpura, primary biliary cirrhosis, scleroderma, vitiligo, pernicious anemia, allergic disease or chronic celiac disease.

The composition may contain a pharmaceutically acceptable carrier, and may further include a pharmaceutically acceptable adjuvant, excipient, or diluent in addition to the carrier.

As used herein, the term "pharmaceutically acceptable" refers to a composition which is physiologically acceptable and generally does not cause an allergic reaction, such as a gastrointestinal disorder and dizziness, or a similar reaction when administered to a human. Examples of the carrier, the excipient, and the diluent include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. Moreover, a filler, an anti-aggregation agent, a lubricant, a wetting agent, a fragrance, an emulsifier, and an antiseptic agent may be further contained.

Furthermore, when the pharmaceutical composition according to one exemplary embodiment of the present invention is administered to a mammal, the pharmaceutical composition can be formulated using methods known in the art to allow rapid, sustained, or delayed release of the active ingredient. Examples of the formulation include powder, a granule, a tablet, an emulsion, a syrup, an aerosol, a soft or hard gelatin capsule, a sterile injectable solution, and a sterile powder form.

The composition according to one exemplary embodiment of the present invention may be administered by various routes such as oral administration and parenteral administration, for example, suppository, transdermal, intravenous, intraperitoneal, intramuscular, intralesional, nasal, or intravertebral administration, and may be administered using an implantation device for sustained release or continuous or repeated release. The administration may be performed once or several times a day within a desired range, and can be performed at an interval such as once a week, twice a week, and once a month, and the duration of administration is also not particularly limited.

The composition according to one exemplary embodiment of the present invention may be formulated in a suitable formulation with a conventional pharmaceutically acceptable carrier. Examples of the pharmaceutically acceptable carrier include carriers for parenteral administration such as water, an appropriate oil, a saline solution, aqueous glucose, and glycol, and a stabilizer and a preservative may be included additionally. Examples of the stabilizer include antioxidants such as sodium hydrogen sulfite, sodium sulfite, or ascorbic acid. Examples of the suitable preservative include benzalkonium chloride, methyl- or propyl-paraben, and chlorobutanol. Moreover, the composition according to an embodiment of the present invention may contain a suspending agent, a solubilizer, a stabilizer, an integerotonic agent, a preservative, an adsorption inhibitor, a surfactant, a diluent, an excipient, a pH adjuster, a painless agent, a buffer agent, an antioxidant, or the like if necessary, depending on the administration method or the formulation. The pharmaceutically acceptable carriers and formulations suitable for the present invention, including those exemplified above, are described in detail in the literature [Remington's Pharmaceutical Sciences, latest edition].

The dosage of the composition to a patient depends on many factors including a height of the patient, a body surface area, an age, a specific compound administered, a gender, a time and a route of administration, general health, and other drugs administered simultaneously. A pharmaceutically active protein can be administered in an amount of 100 ng/body weight (kg) to 10 mg/body weight (kg), more preferably 1 to 500 µg/kg (body weight), and most preferably 5 to 50 µg/kg (body weight), but the dosage can be adjusted in consideration of the abovementioned factors.

In another aspect of the present invention, there is provided a method of suppressing immune response in a subject in need of immunosuppression comprising administering a therapeutically effective amount of the monomeric IL-10 variant protein or the fusion protein to the subject.

The subject may be human or mammal except human, and maybe a patient who has received an organ transplant or a patient with immune-related disease that requires immunosuppression.

In addition, the monomeric IL-10 variant protein or fusion protein of the present invention can be administered in a therapeutically effective amount.

As used herein, the term "therapeutically effective amount" means an amount sufficient to treat a disease at a reasonable benefit/risk ratio applicable to medical treatment, and the effective dose level is the type and severity of the subject, Age, sex, activity of the drug, sensitivity to the drug, time of administration, route of administration and rate of excretion, duration of treatment, factors including concurrent drugs and other factors well known in the medical field. The therapeutically effective amount of the composition of the present invention may be 0.1 mg/kg to 1 g/kg, more preferably 1 mg/kg to 500 mg/kg, but the effective dosage may be adjusted appropriately according to the patient's age, sex and condition.

### EXAMPLES

Hereinafter, the present invention will be described in more detail through Examples and Experimental Examples. However, the present invention is not limited to Examples and Experimental Examples described below, and may be implemented in various other forms, and Examples and Experimental Examples described below are provided to enable the disclosure of the present invention to be complete and to fully convey the scope of the invention to those skilled in the art to which the present invention belongs.

### Example: Preparation of a monomeric human IL-10 variant.

The present inventors have designed various monomeric human IL-10 variants. Specifically, the structure of the IL-10 protein was analyzed, and the minimum linker length was devised to allow the N-terminus and C-terminus of the IL-10 of a single molecule to form a pair, considering that the N-terminus of an IL-10 molecule were paired to the C-terminus of another IL-10 molecule and thus they form a dimer through the pairing. It was expected that a linear distance of 17.3Å was required to bind the N-terminus and C-terminus of a single IL-10 molecule, requiring a linker length of at least 7 a.a. between the N-terminus and C-terminus. In this case, since the N-terminus part and the C-terminus part within a single molecule should be combined, it is expected that the linker that is too long can act as an obstacle to the binding between the both terminus so that 9 a.a. is configured as an optimal linker length and may be designed to include a spacer peptide of up to length of 12 a.a.

In particular, the present inventors determined a human IL-10 variant protein sequence having a configuration shown in Table 1 below and prepared vector constructs comprising polynucleotides encoding various IL-10 variant proteins, respectively, by reacting an NTIG Fc sub-vector, IL-10Vm sub-vector, and a backbone vector (pBispecific Backbone; Genexine, Inc.) in a single tube using Type II restriction enzyme, *Bsa*I and T4 ligase.

**Table 1**

| The structure of the fusion proteins prepared according to embodiments of the present invention | | | | |
|---|---|---|---|---|
| Examples | Name | Structure | linker | spacer |
| Comparative Example 1 | IL-10M | NTIG-linker 1-IL-10V | AAGSGGGGGSGGG GSGGGGS (SEQ ID NO: 2) | n.a. |
| Example 1 | IL-10M-1 | NTIG-linker 1-IL-10Vm (spacer) | AAGSGGGGSGGG GSGGGGS (SEQ ID NO: 2) | GGSGGSGGS (SEQ ID NO: 4) |
| Example 2 | IL-10M-2 | NTIG-linker 1-IL-10Vm (spacer) | GGSGG (SEQ ID NO: 3) | GGSGGSGGS (SEQ ID NO: 4) |
| Comparative Example 2 | IL-10M-3 | NTIG-linker 1-IL-10Vm (spacer) | GGSGG (SEQ ID NO: 3) | GGGSGG (SEQ ID NO: 5) |

As described in the above Table 1, in one embodiment of this invention, an Fc region was linked to the N-terminus of the IL-10 protein, a dimer was formed thereby and the IL-10 variant protein was devised in a form that could facilitate the separation and purification step of the fusion protein. The Fc protein used herein is a modified Fc region (SEQ ID NOs: 8 and 9), consisting of the hinge of IgG1, and CH2 and CH3 which is a hybrid proteins of IgD and IgG4, and the same one described in international patent application PCT/KR2020/006346 was used. For convenience the modified Fc region was referred to as "NTIG". The PCT application is incorporated herein by reference. It was designed to insert a linker peptide consisting of an amino acid sequence of SEQ ID NOs: 2 or 3 between the modified Fc region and the IL-10 variant protein according to an embodiment of the present invention. The IL-10 protein used in the Comparative Example 1 is a variant protein (SEQ ID NO: 10) that suppresses immune activation by substituting isoleucine, the 87^{th} amino acid of the wild-type human IL-10 protein, with alanine, and the Examples 2 and 3 also include the same substitution of the 87^{th} amino acid. Meanwhile, the IL-10 proteins (SEQ ID NOs: 1 and 11) used in the Examples 1 and 2 and Comparative Example 2 have spacer peptides inserted between asparagine (N), which is the 116^{th} amino acid, and lysine (K), which is the 117^{th} amino acid, and in particular, spacer peptides consisting of amino acid represented by SEQ ID NOs: 4 or 5. In particular, the IL-10 protein (SEQ ID NO: 11) comprising a linker peptide having amino acid sequence represented by SEQ ID NO: 5 according to the Comparative Example 2 contains the same monomeric IL-10 protein that Joshepson *et al.* devised (Josephson et al., J. Biol. Chem. 275(18): 13552-13557, 2000). Polynucleotides encoding the components of each fusion protein devised as described above were prepared by PCR amplification and oligonucleotide synthesis, and then prepared a final vector construct by reacting reaction mixture in a single tube after subcloning them into NTIG sub-vector and IL-10Vm sub-vector and a backbone vector (pBispecific vector, Genexine, Inc., Korea) using *Bsa*I restriction enzyme and T4 ligase. The vector constructs prepared as described above were temporarily expressed using an ExpiCHO kit manufactured by Thermo Fisher. Specifically, after mixing the above-prepared vector constructs and the ExpiFectamine reagent contained in the kit with the ExpiCHO-S cell, incubation was performed for 1 day in an incubator with conditions of 8% CO₂ and 37°C, and the temperature was lowered to 32°C to 7 days.

And then, protein A capture purification was performed, and it was confirmed whether the candidate materials were purified through SDS-PAGE analysis under non-reduction and reduction conditions, and formulation was performed with a formulation buffer considering the pI value of the candidate material. The candidate materials whose formulation was completed were quantified using Nano Drop, and the final purity was determined using SEC-HPLC. FIGs. 2A and 2B represent purity of proteins using SDS-PAGE and SEC-HPLC for culture and purified materials of IL-10M. FIGs. 2C and 2D represent purity of proteins using SDS-PAGE and SEC-HPLC for culture and purified materials of IL-10M-1 and IL-10M-2, respectively. FIGs. 2E and 2F represent purity of candidate materials using SDS-PAGE and SEC-HPLC for culture and purified materials of IL-10M-3.

**Table 2**

| Results of production of fusion proteins according to embodiments of the present invention | | | | |
|---|---|---|---|---|
| Examples | Conc. of protein (mg/ml) | Volume (ml) | Total amount of protein (mg) | Purity (%) |
| Comparative Example 1 | 5.0 | 3 | 15.2 | 57.5 |
| Example 1 | 4.3 | 6.2 | 26.66 | 82.7 |
| Example 2 | 3.64 | 6.2 | 22.56 | 78.1 |
| Comparative Example 2 | 18.5 | 3 | 55.64 | 70.5 |

As a result, as shown in Table 2 and FIGs. 2A to 2F, when non-monomeric IL-10 was produced (Comparative Example 1), yield was low and lowest purity was shown, and when monomeric IL-10 was produced (Examples 1 and 2 and Comparative Example 2), protein content and purity were significantly increased. Meanwhile, although in the Comparative Example 2, the protein content was the highest, in terms of purity, the protein of the Example 1 of the present invention showed the highest purity of 82.7%.

### Example 3: Preparation of FcεRI--Fc-IL-10Vm

### 3-1: Transient expression

The present inventors devised a fusion protein in which FcεRIα, a receptor specifically binding to IgE, and the monomeric IL-10 variant proteins (IL-10Vm) of the Example 1 and Comparative Example 2 are linked to Fc proteins.

Specifically, the present inventors prepared polynucleotides encoding fusion proteins having configurations shown in the below Table 3 using oligonucleotide synthesis and PCR amplification, and prepared a final vector construct by reacting reaction mixture in a single tube after subcloning them into FcεRIα sub-vector, NTIG Fc sub-vector, IL-10Vm sub-vector and a backbone vector in the same manner as in the Example 1.

**Table 3**

| Components | Example 3 (SEQ ID Nos) | Comparative Example 3 (SEQ ID Nos) |
|---|---|---|
| Signal sequence | 32 | 32 |
| FcεRIα | 33 | 33 |
| Linker 1 | 31 | 31 |
| Fc | 8 | 8 |
| Linker 2 | 2 | 3 |
| IL-10Vm | 1 | 11 |

The present inventors designated the fusion protein, FcεRIα-Fc-IL-10Vm of the Example 3 as "PG075-8", and FcεRIα-Fc-IL-10Vm of the Comparative Example 3 as "PG075-9". The vector constructs prepared as described above were transiently expressed using ExpiCHO kit manufactured by Thermo Fisher. Specifically, after mixing the above-prepared vector construct and the ExpiFectamine reagent contained in the kit with ExpiCHO-S cells in the kit, the mixture was incubated at 8% CO₂ and 37°C for 1 day. Thereafter, the temperature was lowered to 32°C and cultured on a 250mL scale until the 7^{th} day.

And then, protein A capture purification was performed, and it was confirmed whether the candidate materials were purified through SDS-PAGE analysis under non-reduction and reduction conditions. And then, formulation was performed with a formulation buffer considering the pI value of the candidate materials. The formulated candidate materials were quantified using Nano Drop, and the final purity was confirmed using SEC-HPLC. FIGs. 3A and 3B represent purity of candidate materials using SDS-PAGE and SEC-HPLC for culture and purified materials of FcεRIα-Fc-IL-10Vm (PG075-8) according to the Example 3, respectively. FIGs. 3C and 3D represent purity of candidate materials using SDS-PAGE and SEC-HPLC for culture and purified materials of FcεRIα-Fc-IL-10Vm (PG075-9) of the Comparative Example 3, respectively.

As shown in FIGs. 3A to 3D, it was confirmed that the IL-10Vm protein according to an embodiment of the present invention exhibits significant high yield and purity when it was expressed as a fusion protein linked to FcεRIα which is a an API (active pharmaceutical ingredient), whereas yield and purity of IL-10 variant protein was decreased significantly when the prior monomeric IL-10 variant protein of the Comparative Example 2 was applied. Accordingly, the present inventors used the IL-10Vm variant protein according to the Example 1 of the present invention in order to establish stable cell lines for the production of the fusion protein in which therapeutically active proteins as APIs are linked to IL-10Vm protein and to prepare the candidate materials at laboratory-scale.

### 3-2: Establishment and production of stable cell lines

Accordingly, the present inventors inserted the gene construction encoding the fusion protein constructed in the Example 3-1 into a pAD15 expression vector (WO2015/009052A) and then transfected it using a Neon-transfection system (CHODG44) cell. As the first screening process, HT screening was performed using 10% dFBS (Gibco, USA, 30067-334), MEMα (Gibco, 12561, USA, Cat No. 12561-049), and HT⁺ (Gibco, USA, 11067-030) medium without HT (5-hydroxypypamine). Subsequently, methotrexate (MTX) amplification was performed using HT-selected clones in order to amplify the expression gene using a DHFR (dihydrofolate reductase) system. MTX amplification was performed in the form of a mini-pool on the plate to isolate high productivity clones, and after screening for one species whose amplification was confirmed, limiting dilution cloning (LDC) (96 wells, 30 plates) was performed to isolate the final cell line.

The final isolated cell line was incubated 700 ml in the hyCellCHO medium, and the purified proteins were identified by performing Protein A purification on the culture medium obtained on the 5^{th} day of cultivation, and yield and purity of the purified proteins were analyzed through SDS-PAGE and SEC-HPLC.

Consequently, as confirmed in FIGs. 3E and 3F, it was confirmed that the fusion protein according to an embodiment of the present invention was normally expressed in the established stable cell line.

### Experimental Example 1: Mixed lymphocyte response analysis

The present inventors analyzed the mixed lymphocyte reaction using whole blood provided from a plurality of donors in order to confirm the immunosuppressive activity of the fusion protein prepared in the examples.

Specifically, the present inventors separated peripheral blood mononuclear cells (PBMC) by centrifuging the whole blood provided from 10 donors by Ficoll gradient centrifugation. After counting the number of separated monocytes, the cells were mixed, and cell stocks were prepared with 5×10⁶ cells per vial (1 mL).

Then, the cells received from the donors and cells of the recipients were thawed, the number of cells was counted, and then resuspended at a concentration of 1×10⁶ cells/ml. After separating the cells of recipients into reactive cells and stimulating cells, the recipient's stimulating cells and donor cells were stimulated by irradiating 3,000 rad gamma rays. Then, the recipient's reactive cells were stained with cell trace violet (V450) and the recipient's stimulating cells and donor cells were stained with cell trace red (APC). 1×10⁵ of reactive cells from the donors and 1×10⁵ of reactive cells from the recipients were added to 200 µl of RPMI medium supplemented with 10% FBS and mixed. The mixed cells were cultured for 6 days at 37°C and 5% CO₂. The fusion proteins of the Comparative Examples 1 and 2 and Examples 1 and 2 were treated at a concentration of 0.5 µM, respectively. After completion of the culture, FACS analysis was performed. Antibodies used in FACS analysis are BV650-conjugated anti-CD3 antibodies, PE (phycoerythrin)-conjugated anti-PD1-antibodies, PE-TR-conjugated anti-CD-14 antibodies, PE-TR-conjugated anti-CD19 antibodies, PerCp-conjugated anti-CD4 antibodies, Cy5.5-conjugated CD4 antibodies, APC (allophycocyanine)-conjugated anti-CD8 antibodies, H7-conjugated anti-CD8 antibodies.

As a result of the FACS analysis, when reactive cells from the recipients and stimulating cells from the recipients were reacted (autologous, abbreviated as 'Auto' hereinafter), no proliferation of CD4⁺ T cells and CD8⁺ T cells in the recipients was found, whereas when the reactive cells from the recipients and stimulating cells from the donors were reacted (allogenic, abbreviated as "Allo") proliferation of CD4⁺ T cells and CD8⁺ T cells in the recipients was confirmed. As shown in FIGs. 4A to 4D, although there was no significant difference, in the case of the dimeric IL-10 fusion protein of the Comparative Example 1, the CD4⁺ proliferation inhibitory activity was most strongly shown, whereas in the case of the fusion protein including the conventional monomeric IL-10 of the Comparative Example 2, there was no difference from the negative control group. On the other hand, the fusion protein including monomeric IL-10 variant protein according to the Examples 1 and 2 of the present invention exhibited better immunosuppressive activity compared than that of the Comparative Example 2. Similarly, CD8⁺ T cell proliferation inhibitory activity was also highest in the dimeric IL-10 fusion protein of the Comparative Example 1, and the monomeric IL-10 fusion protein according to Examples 1 and 2 of the present invention showed lower CD8⁺ T cell proliferation inhibitory activity than that of the Comparative Example 2.

### Experimental Example 2: Mast cell proliferation analysis

The present inventors have attempted to investigate the immune-stimulatory activity of the monomeric IL-10 fusion protein of the present invention.

In particular, bone marrow-derived mast cells (BMMC) of 5×10³ cells/100 µl/well were seeded in a 96-well plate containing an RPMI medium containing 10% FBS, 1% antibiotics, rmSCF 20 ng/ml and rmIL-3 10 ng/ml, and treated the cells with the recombinant human IL-10 and IL-10 fusion protein according to the Comparative Examples 1 and 2 and Examples 1 and 2 after diluting them at an appropriate concentration. Subsequently, 20 µl of MTS reagents were dispensed into 96 well plates to measure the degree of proliferation of mast cells, and then the 96 well plates were put into a CO₂ incubator at a temperature of 37°C and reacted for 2 hours, and then absorbance was measured at 595 nm using a microplate reader.

**Table 4**

| | | | | | | | |
|---|---|---|---|---|---|---|---|
| Conc. (pM) | 0 | 0.5 | 2.9 | 14 | 72 | 360 | 2000 |
| rhIL-10 | 1 | 1.01 | 1.06 | 1.21 | 1.37 | 1.55 | 1.63 |
| Comparative Example 1 | 1 | 1.00 | 1.00 | 0.98 | 1.05 | 1.20 | 1.26 |
| Example 1 | 1 | 1.06 | 1.03 | 1.02 | 1.03 | 1.02 | 1.00 |
| Example 2 | 1 | 1.00 | 1.00 | 1.01 | 0.97 | 1.02 | 1.01 |
| Comparative Example 2 | 1 | 0.99 | 1.03 | 1.18 | 1.20 | 1.25 | 1.40 |

As a result, as shown in FIG. 5A and Table 4, the proliferation of mast cells treated with the rhIL-10 (positive control) was the strongest, and the proliferation of mast cells treated with the IL-10 fusion proteins of Comparative Examples 1 and 2 were lower than that of cells treated with rhIL-10, but were found to have a certain degree of mast cell proliferation activity. On the other hand, the IL-10 fusion proteins according to the Examples 1 and 2 of the present invention showed little mast cell proliferation activity. This is a result showing that the IL-10 variant protein according to the present invention has completely suppressed immune activation property among the dual activities of IL-10. However, in the case of the experimental results, it can be interpreted that the fusion proteins according to the Examples 1 and 2 of the present invention were expressed in a form with no activity, so the present inventors performed the same analysis by increasing the treated concentration only for rhIL-10, the fusion protein according to the Example 1, and Comparative Example 1.

**Table 5**

| | | | | | | | | | | |
|---|---|---|---|---|---|---|---|---|---|---|
| Conc. (nM) | 0 | 0.004 | 0.02 | 0.04 | 0.16 | 0.63 | 2.5 | 10 | 40 | 100 |
| rhIL-10 | 1 | 1 | 1 | 1.19 | 1.42 | 1.58 | 1.71 | 1.79 | 1.78 | 1.78 |
| Comparative Example 1 | 1 | 0.99 | 0.99 | 0.97 | 1 | 1.19 | 1.35 | 1.36 | 1.35 | 1.35 |
| Example 1 | 1 | 0.97 | 0.97 | 0.99 | 1.01 | 1.01 | 1 | 1.06 | 1.02 | 1.2 |

As a result, as shown in Table 5 and FIG. 5B, the fusion protein according to the Example 1 of the present invention showed limited mast cell proliferation activity only at very high concentrations of 100 nM. However, since the concentration is significantly higher than the dose used for *in vivo* administration, it is expected that there will be little immune-stimulating effect such as mast cell proliferation when administered *in vivo.*

### Experimental Example 3: Immunosuppressive Activity Analysis

Following the analysis of mast cell proliferation, the effect of IL-10 fusion protein on the reduction of TNF-α-secretion activity in mast cells, i.e., immunosuppressive activity, was investigated.

### 3-1: Analysis of TNF-α-secretion inhibitory activity in mast cells

To this end, specifically, bone marrow-derived mast cells (BMMC) of 1×10⁴ cells/50 µl/well were seeded in a 96-well plate containing an RPMI medium containing 10% FBS, 1% antibiotics, rmSCF 20 ng/ml and rmIL-3 10 ng/ml, and treated the cells with the recombinant human IL-10 and IL-10 fusion protein according to the Comparative Examples 1 and 2 and Examples 1 and 2 were diluted to appropriate concentration. And then, anti-DNP IgE diluted to 3 µg/mL and 50 µl was added to a 96-well plate and incubated for 24 hours at 37°C and 5% CO₂ conditions. Subsequently, 50 µl of DNP-BSA (Antigen) diluted to 400 ng/mL was added to 96 well plates, and then reacted overnight at 37°C and 5% CO₂ conditions. Then, centrifugation was performed at 4°C at a speed of 1,500 rpm for 5 minutes, the supernatant 150 µl recovered was dispensed in a new 96-well plate, and the concentration of TNF-α was measured using a TNF-α ELISA kit (Biolegend, USA).

**Table 6**

| | | | | | |
|---|---|---|---|---|---|
| Conc. (n) | 0 | 0.31 | 1.25 | 5 | 20 |
| Comparative Example 1 | 0% | 27% | 37% | 51% | 51% |

**Table 7**

| | | | | | |
|---|---|---|---|---|---|
| Conc. (n) | 0 | 1.56 | 6 | 25 | 100 |
| Example 1 | 0% | 6.3% | 27% | 43% | 45% |
| Example 3 | 0% | 58% | 65% | 80% | 83% |
| Example 2 | 0% | 21% | 33.6% | 39% | 46% |
| Comparative Example 2 | 0% | 13% | 31% | 45% | 49% |

As a result, in Comparative Example 1 (IL-10M), concentration-dependent TNF-α inhibitory activity was shown, as shown in Tables 6 and 7 and FIGs. 6A to 6C. At this time, IL-10M-1 in the Example 1 and IL-10M-2 in Example 2 of the present invention and IL-10M-3 in the Comparative Example 2 showed a concentration-dependent TNF-α secretion inhibitory effect similar to that in the Comparative Example 1, but their concentration was 5 times higher than in the Comparative Example 1. Moreover, PG075-8 according to an embodiment of the present invention exhibits very excellent TNF-α secretion inhibitory activity even at lower concentrations, confirming that effective blocking of IgE is possible. Summarizing the above results, it can be seen that the IL-10 variant protein according to an embodiment of the present invention had lower immunosuppressive activity than the dimeric IL-10 variant protein, but was most effective in inhibiting immune-stimulating activities, one of the dual activities of IL-10, and is the most advantageous in terms of yield and purity of proteins.

In particular, it was confirmed that the IL-10 variant protein according to an embodiment of the present invention exhibited concentration-dependent TNF-α secretion inhibitory activity compared to the IL-10 variant protein of the Comparative Example 2, which is similarly expressed in a monomeric form, and it had a significant inhibitory activity of allergy reaction when it is used as a fusion protein in which a FcεR1α is linked thereto (Example 3).

### 3-2: Analysis of LPS-induced TNF-α secretion inhibitory activity in macrophages

The inventors investigated whether TNF-α secretion inhibitory activity of PG075-8 protein according to an embodiment of the present invention in macrophages which are immune cells closely related to TNF-α-mediated inflammatory reaction, confirming that TNF-α secretion inhibitory activity of PG075-8 protein according to an embodiment of the present invention in mast cells.

To this end, 100 µL of RAW264.7 cells prepared at a concentration of 5×10⁵ cells/mL were dispensed into 96 well plates, and then cultured for 12 hours in a 5% CO₂ and 37°C incubator. RAW264.7 cells in culture were treated with the PG075-8 protein according to an embodiment of the present invention after diluting sequentially the protein at an appropriate concentration. In this case, TNF-α expression was induced by treating 100 µL of LPS (400 ng/mL) on 96 well plates. Then, 150 µL of supernatant was separated after 12 hours of incubation at 37°C and 5% CO₂ incubator, and the amount of TNF-α expression level contained in the supernatant was measured in the same manner as in the Experimental Example 3-1 (Table 8 and FIG. 6D).

**Table 8**

| TNF-α secretion inhibititory rate of the fusion protein (PG075-8) according to an embodiment of the present invention in macrophages | | | | | | | | | | |
|---|---|---|---|---|---|---|---|---|---|---|
| | Conc. (nM) | | | | | | | | | IC₅₀ (nM) |
| | 0.019 | 0.096 | 0.48 | 2.4 | 12 | 60 | 100 | 300 | 1507 | |
| TNF-α secretion inhibitory tate (%) | 7% | 13% | 10% | 14% | 22% | 31% | 38% | 40% | 42% | 10.42 |

Consequently, as shown in Table 8 and FIG. 6D, PG075-8 fusion protein according to an embodiment of the present invention inhibited expression of TNF-α in macrophages depending on treated concentration.

### Experimental Example 4: Analysis of binding affinity to IL-10 receptors

Based on the experimental results of the Experimental Examples 1 to 3, the present inventors analyzed binding affinity of the fusion proteins of the Comparative Example 1 and Example 1 of the present invention to IL-10 receptor 1 (IL-10R1) through bio-layer interferometry (BLI) analysis. At this time, as controls, a fusion protein in which FcεRIα is not connected and a dimeric IL-10 variant protein (IL-10V) having amino acid sequence represented by SEQ ID NO: 10 whose immuno-stimulating activity is inhibited by substituting isoleucine, 87^{th} amino acid of wild type IL-10 protein, with alanine is linked to a hybrid Fc region, and a fusion protein (NTIG-IL-10Vm) in which a monomeric IL-10 variant protein having an inserted spacer peptide (GGSGGSGGS) between the 116^{th} amino acid, asparagine (N) and the 117^{th} amino acid, lysine (K) were used. The NTIG-IL-10Vm is the same as PG075-8 according to an embodiment of the present invention, except that FcεRIα is not connected.

To this end, first of all, these inventors attached IL-10R His-tag protein to a 96-well plate using the Dip and Read^{™} Amine Reactive 2n^{d} Generation (AR2G) Reagent Kit (forteBio, Cat No. 18-5092). Specifically, after dispensing 200 µl of D.W. in a 96-well plate, an amine biosensor included in the kit was inserted and hydrated for 10 minutes. Subsequently, after dispensing 200 µl of D.W. additionally to the plate, EDC:NHS was mixed in a 1:1 ratio in a volume corresponding to 1/20 of the required sample, then diluted in D.W. and 200 µl was dispensed into the 96-well plate. Subsequently, IL-10R His-tag protein was diluted to 10 µg/ml in 10 mM acetic acid solution (pH 5.0), and 200 µl was dispensed into the 96-well plate. Then, 200 µl of 1 M ethanolamine was added to the 96-well plate, and the biosensor plate and the sample plate were inserted into the Octet^{®} K2 BLI analyzer and signals were measured. After the measurement was completed, 200 µl of the 1x Kinetics buffer was added to the sample plate, and then a baseline was determined. Subsequently, the NTIG-IL-10 fusion protein prepared in the above Example was diluted in a 1x Kinetic buffer solution at various concentrations (0, 62.5, 125, 250, 500, and 1,000 nM), then dispensed to the sample plate at 200 µl, and then BLI analysis was performed to measure binding affinity using Octet^{®} K2 BLI analyzer.

**Table 9**

| Parameter | Comparative Example 1 | Example 1 | Example 3 (PG075-8) |
|---|---|---|---|
| K_{D} (nM) | 11.1±0.9 | 29.2±8.85 | < 0.001 20 |
| Kₒₙ (1/Ms) | 71100±6670 | 20550±2312 | 11,500±980 |
| K_{dis} (1/s) | 0.0008 | 0.0006 | < 1.0E-07 |
| R² | 0.97 | 0.97 | 0.98 |

As a result, as shown in FIG. 7 and Table 9, the fusion protein (NTIG-IL-10Vm) according to an embodiment of the present invention has a K_{D} value of 29.2 nM, which is about three times compared with that of the monomeric IL-10 fusion protein (NTIG-IL-10V) of the Comparative Example 1 (11.1 nM). Therefore, binding affinity of NTIG-IL-10Vm to IL-10R was found to be somewhat lower than that of the monomeric IL-10 fusion protein of the Comparative Example 1. The results of previous studies also showed that monomeric IL-10 had lower binding affinity to IL-10R compared to dimeric IL-10 protein, so it is fully predictable. On the other hand, the fusion protein of the Example 1 of the present invention (FcεRIα-Fc-IL-10Vm, PG075-8) had a K_{D} value of less than 0.001 nM, indicating a higher binding affinity to IL-10R. This is a result showing that the monomeric IL-10 variant has sufficient binding affinity to IL-10R despite being linked to FcεRIα, an API.

### Experimental Example 5: IgE binding activity analysis

The present inventors analyzed binding affinity of the fusion protein (FcεRIα-Fc-IL-10Vm) prepared in Example 3 to mouse IgE and human IgE through biolayer interferometry (BLI) analysis.

To this end, first of all, the present inventors attached FcεRIα-Fc fusion protein not containing IL-10Vm (control), and the fusion protein prepared in the Example 3 to 96-well plates, respectively using Dip and Read^{™} Amine Reactive 2^{nd} Generation (AR2G) Reagent Kit (forteBio, Cat No. 18-5092). Specifically, after dispensing 200 µl of D.W. to the 96-well plate, the amine biosensor included in the kit was inserted and hydrated for 10 minutes. Then, after dispensing additionally 200 µl of D.W. to the 96-well plate, EDC:NHS was mixed in a 1:1 ratio in a volume corresponding to 1/20 of the required sample then diluted in D.W. and 200 µl was dispensed into the 96-well plate. Subsequently, the FcεRIα-Fc fusion protein and the FcεRIα-Fc-IL-10Vm fusion protein was diluted to 10 µg/ml in 10 mM acetic acid solution (pH 5.0), and 200 µl was dispensed into the 96-well plate. Then, 200 µl of 1 M ethanolamine was added to the 96-well plate, and the biosensor plate and the sample plate were inserted into the Octet^{®} K2 BLI analyzer and signals were measured. After the measurement was completed, 200 µl of the 1x Kinetics buffer was added to the sample plate, and then a baseline was determined. Subsequently, anti-DNP mouse IgE antibodies (Sigma, USA) were diluted in a 1x Kinetic buffer solution at various concentrations (50 pM to 3.125 nM), then dispensed to the sample plate at 200 µl, and then BLI analysis was performed to measure binding affinity using Octet^{®} K2 BLI analyzer.

**Table 10**

| | FcεRIα-Fc | | | PG075-8 (FcεRIα-Fc-IL-10Vm) | | |
|---|---|---|---|---|---|---|
| | 1st | 2nd | 3rd | 1st | 2nd | 3rd |
| Avg. K_{D} (nM) | 0.364 | 0.478 | 0.940 | 0.224 | 0.282 | 0.346 |
| | Avg.: 0.594 | | | Avg.: 0.284 | | |
| Avg. Kon (1/Ms) | 1.23×10⁵ | 1.49×10⁵ | 1.66×10⁵ | 1.52×10⁵ | 1.44×10⁵ | 1.39×10⁵ |
| Avg. K_{dis}(1/s) | 4.48×10⁻⁵ | 7.10×10⁻⁵ | 1.56×10⁻⁴ | 3.40×10⁻⁵ | 4.06×10⁻⁵ | 4.82×10⁻⁵ |
| Avg. R² | 0.996 | 0.999 | 0.997 | 0.999 | 0.999 | 0.999 |

As a result, as shown in FIG. 8A and Table 10, the K_{D} value of the fusion protein (FcεRIα-Fc-IL-10Vm, PG075-8) according to an embodiment of the present invention is 0.284 nM, which is lower than that of the FcεRIα-Fc fusion protein which is a control. Although it was found to be somewhat low than the control, the difference is not significant. Thus, it was confirmed that the binding affinity of the fusion protein according to an embodiment of the present invention to IgE was not deceased by the addition of IL-10 protein. Together with the above results, the present inventors analyzed binding affinity of the fusion protein (PG075-8) of the Example 3 and human IgE using the method described above, in order to measure binding affinity of human IgE and human FcεRIα used in the present invention.

**Table 11**

| | PG075-8 (FcεRIα-Fc-IL-10Vm) |
|---|---|
| Avg. K_{D} (nM) | 0.346 |
| Avg. Kon (1/Ms) | 2.19×10⁵ |
| Avg. Kdis (1/s) | 7.59×10⁻⁵ |
| Avg. R² | 0.9955 |

As a result, as shown in FIG. 8B and Table 11, the fusion protein according to an embodiment of the present invention exhibited a similar level of binding affinity to human IgE as that of mouse IgE.

### Experimental Example 6: Pharmacokinetics analysis

The present inventors performed pharmacokinetic analysis to confirm how stable the fusion protein of the present invention is when administered *in vivo.*

Specifically, the fusion protein (PG075-8) according to an embodiment of the present invention was administered to 3 SD rats in each group by intravenous injection, intraperitoneal injection, intramuscular injection, and subcutaneous injection at a dose of 1 mg/Kg body weight, respectively. After administration in volumes of 1.0 mL/kg, 1.0 mL/kg, 0.4 mL/kg and 1.0 mL/kg, respectively, at 0 min, 5 min, 1 hours, 5 hours, 10 hours, 24 hours, 48 hours, 72 hours, 120 hours, 168 hours, 240 hours and 336 hours, in case of intravenous injection, and in the case of other administration methods (intraperitoneal injection, intramuscular injection, and subcutaneous injection) at the same time as the intravenous injection, except for measurement after 5 minutes, serum was collected and the fusion protein was quantified using human IgG4 Fc ELISA.

As a result, as shown in FIG. 9, the fusion protein according to an embodiment of the present invention showed a modest decrease over time regardless of the route of administration, indicating that the fusion protein according to an embodiment of the present invention is stably maintained in the body for a considerable period of time.

### Experimental Example 7: Hematotoxicitv analysis

Conventional recombinant IL-10 has a side effect of anemia symptoms due to decreasing concentration of hemoglobin in blood and thrombocytopenia due to the decrease of concentration of platelets in blood when the administration dose is increased or repeated administration is applied (Tilg et al., J. Immunol. 164(4): 2204-2209, 2002; Fedorak et al., Gastroendocrinol. 119(6): 1473-1482, 2000). Accordingly, the present inventors investigated whether the fusion protein according to an embodiment of the present invention would exhibit such side effects.

To this end, specifically, the present inventors prepared 3 female ICR mice in each group, and administered PG075-8 at doses of 0, 50, 150 and 300 mg/kg to each group, and collected blood on the 11^{th} day after drug administration. Then, the present inventors counted the white blood cell differential count, total red blood cells (RBCs), and total platelets using an automatic hematology analyzer (XN-V, SYSMEX, JAPAN). As a result, as shown in FIG. 10, there was no effect on the number of blood cells or the like when PG075-8 was administered.

### Experimental Example 8: Analysis of efficacy on food allergy

The present inventors investigated whether symptom of diarrhea which is a representative symptom allergy that occur when OVA is administrated orally to experimental animals sensitized with albumin (OVA) was alleviated by the administration of the fusion protein PG075-8 according to an embodiment of the present invention to the experimental animals in order to confirm of the therapeutic effect of the fusion protein on allergy diseases.

Specifically, Balb/c mice (Koatech, Korea) were sensitized by intraperitoneally administering a solution of 50 µg of OVA (ovalbumin) and 1 mg of Alum twice at an interval of 14 days. Then, on days 28, 30, 32, 34, and 36, 50 mg of OVA was orally administered at 2-day intervals over a total of 5 times to induce food allergy in the intestine. In the process, the drugs constituting each group were administered on the 31^{st} day. Phosphate buffer solution (PBS) in the first group (control group), IgE_{TRAP} (5 mg/kg) in the second group, and PG075-8 (7.3 mg/kg) according to an embodiment of the present invention in the third group, respectively, was administered after calculating the number of moles, respectively. Diarrhea occurrence was scored while oral administration of OVA was administered 5 times in total. On day 37, the mice were sacrificed and autopsied. Then, the number of mast cells in the small intestine, blood IgE concentration, and concentration of degranulation enzyme concentration (MCPT-1, Mast cell protease-1) in blood mast cells in the mice of each group were analyzed. As a result, as shown in FIG. 11, it was confirmed superior effect of alleviating food allergy in the mice belonging to the group administered with PG075-8 according to an embodiment of the present invention compared with the control group and the IgE_{TRAP} group.

Accordingly, the fusion protein which IL-10Vm and FcεRIα as another fusion partner are applied according to an embodiment of the present invention inhibits the activation of the immune cells and their functions, particularly the secretion of anti-inflammatory cytokines, antigen presentation activity, etc. Thus, it can be used for the treatment of various immune-related diseases caused by overactivated immune function, for example, allergic diseases such as atopic disease, food allergy, chronic spontaneous urticaria, asthma, and the like.

The present invention has been described with reference to the above-described embodiments and experimental examples, but these are merely exemplary, and those of ordinary skill in the art will appreciate that various modifications and equivalent other embodiments are possible therefrom. Therefore, the true technical protection scope of the present invention should be determined by the technical spirit of the appended claims.

## Claims

1. A monomeric IL-10 variant protein in which a spacer peptide with a length of 6 to 12 a.a. are inserted between asparagine, the 116^{th} amino acid, and lysine, the 117^{th} amino acid, based on the mature form of the human Interleukin-10 (IL-10).

2. The monomeric IL-10 variant protein according to claim 1, wherein the protein consists of amino acid sequence represented by SEQ ID NOs: 1 or 39.

3. A fusion protein comprising a monomeric IL-10 variant protein in which a spacer peptide with a length of 6 to 12 a.a. are inserted between asparagine, the 116^{th} amino acid, and lysine, the 117^{th} amino acid, based on the mature form of the human Interleukin-10 (IL-10)

4. The fusion protein according to claim 3, wherein the monomeric IL-10 variant protein consists of amino acid sequence represented by SEQ ID NOs: 1 or 39.

5. The fusion protein according to claim 3, further including one or more fusion partner proteins that perform different functions.

6. The fusion protein according to claim 5, wherein the fusion partner protein is an antibody specifically binding to a target protein, an antigen-binding fragment of the antibody, an antibody mimetic specifically binding to the target protein, an antibody Fc region, or an antibody Fc region receptor, an extracellular domain of the antibody Fc region receptor, a dimerization domain, a cytokine or an immunomodulatory peptide.

7. The fusion protein according to claim 6, wherein the antibody Fc region consists of any one amino acid sequence among SEQ ID NOs: 6 to 9.

8. The fusion protein according to claim 6, wherein the antibody Fc region receptor is alpha subunit of IgE Fc receptor or an extracellular domain of the alpha subunit.

9. The fusion protein according to claim 6, wherein the antigen-binding fragment of the antibody is Fab, F(ab')₂, Fab', scFv, diabody, triabody, sdAb (single domain antibody), V_{NAR} or V_{H}H.

10. The fusion protein according to claim 6, wherein the antibody mimetic is affibody, affilin, affimer, affitin, alphabody, anticalin, avimer, DARPin, Fynomer, Kunitz domain peptide, monobody, repebody, VLR, or nanoCLAMP

11. The fusion protein according to claim 6, wherein the antibody Fc region is an Fc region of IgG, IgA, IgD or IgM or a hybrid Fc in which two or more domains of Fc regions of the above-described Ig subclasses are mixed.

12. The fusion protein according to claim 6, wherein the dimerization domain is a hinge domain of antibody, LIM/double zinc-finger motif, RAG1 domain, HAT dimerization domain, TRFH dimerization domain, Stat3 dimerization domain, or LFB1/HNF1 dimerization domain.

13. The fusion protein according to claim 6, wherein the cytokine is IL-4, IL-6, IL-1α or TGF-β.

14. The fusion protein according to claim 6, wherein the immunomodulatory peptide is PD-1L or CTLA-4 (CD152).

15. A polynucleotide encoding the monomeric IL-10 variant protein according to claims 1 or 2 or the fusion protein according to any one among claims 3 to 14.

16. A vector comprising the polynucleotide of claim 15.

17. A pharmaceutical composition for immunosuppression comprising the monomeric IL-10 variant protein according to claims 1 or 2 or the fusion protein according to any one among claims 3 to 14 as an active ingredient.

18. A pharmaceutical composition for the treatment of an immune-related disease comprising the monomeric IL-10 variant protein according to claims 1 or 2 or the fusion protein according to any one among claims 3 to 14 as an active ingredient.

19. The pharmaceutical composition according to claim 18, wherein the immune-related disease is type 1 diabetes, alopecia areata, anti-phospholipid antibody syndrome, rheumatoid arthritis, psoriasis or psoriatic arthritis, multiple sclerosis, systemic lupus erythematosus, inflammatory bowel disease, Addison's disease, Graves' disease, Sjögren's syndrome, Guillain-Barre syndrome, Hashimoto's thyroiditis, Myasthenia gravis, inflammatory myophathy, autoimmune vasculitis, autoimmune hepatitis, hemorrhagic anemia, idiopathic thrombocytopenic purpura, primary biliary cirrhosis, scleroderma, vitiligo, pernicious anemia, allergic disease or chronic celiac disease.
